# FASCICULE DE BREVET EUROPEEN

(11) **EP 1 220 950 B1**
(45) Date de publication et mention de la délivrance du brevet: **31.03.2004**
(21) Numéro de dépôt: 00962602.9
(22) Date de dépôt: 12.09.2000
(51) Int. Cl.: C12Q 1/68, C12N 15/10

(54) **MARQUEURS GENETIQUES DE LA TOXICITE, PREPARATION ET UTILISATION**
GENETISCHE TOXIZITÄTSMARKER, HERSTELLUNG UND VERWENDUNG
GENETIC MARKERS OF TOXICITY, PREPARATION AND USES THEREOF

(30) Priorité: 13.09.1999 FR 9911405
(43) Date de publication de la demande: 10.07.2002
(73) Titulaire: Exonhit Therapeutics S.A., 75013 Paris (FR)
(72) Inventeur: TOCQUE, Bruno, F-92400 Courbevoie (FR); BRACCO, Laurent, F-75014 Paris (FR); SCHWEIGHOFFER, Fabien, F-94300 Vincennes (FR)
(74) Mandataire: Becker, Philippe
(86) Numéro de dépôt international: PCT/FR2000/002503
(87) Numéro de publication internationale: WO 2001/020029

(56) Documents cités:
- WO-A-00/12760
- WO-A-97/13877
- WO-A-99/10529
- WO-A-99/42606
- FR-A- 2 775 984
- SCHENA M ET AL: "PARALLEL HUMAN GENOME ANALYSIS: MICROARRAY-BASED EXPRESSION MONITORING OF 1000 GENES" PROCEEDINGS OF THE NATIONAL ACADEMY OF SCIENCES OF USA,US,NATIONAL ACADEMY OF SCIENCE. WASHINGTON, vol. 93, no. 20, 1 octobre 1996 (1996-10-01), pages 10614-10619, XP002912238 ISSN: 0027-8424
- CICHON S. ET AL.,: "Pharmacogenetics of schizophrenia" AM. J. MEDICAL GENETICS, vol. 97, mars 2000 (2000-03), pages 98-106, XP002169375

## Description

La présente invention se rapporte aux domaines techniques de la biotechnologie, de la médecine, de la biologie et de la biochimie. Ses applications concernent les domaines de la santé humaine, animale et végétale. Plus particulièrement, l'invention décrit de nouvelles méthodes qui permettent de déterminer le potentiel toxique de composés tests ou de prédire la sensibilité ou la réponse de patients à des composés, ainsi que des outils et kits pour la mise en oeuvre de ces méthodes. L'invention décrit également des méthodes pour obtenir des séquences d'acides nucléiques utilisables comme marqueurs génétiques de la toxicité. L'invention est particulièrement utile dans l'industrie pharmaceutique, pour l'analyse du profil toxique de molécules qui pourront entrer en développement pharmaceutique et /ou dans des compositions pharmaceutiques.

Les problèmes de toxicité constituent la raison majeure d'abandon de molécules à visée thérapeutique au cours du développement pré-clinique et clinique. A notre connaissance, il n'existe à ce jour aucun test permettant de diagnostiquer rapidement la toxicité de molécules chez l'homme. Les autorités réglementaires demandent cependant qu'une nouvelle molécule candidate soit soumise à une série de tests de toxicité, mutagénicité, carcinogénicité et tératogénicité sur animaux et à des essais cliniques chez l'homme. Ces tests sont longs, coûteux et ne sont que partiellement satisfaisants. Ainsi, la toxicité chez l'animal est loin de refléter la toxicité chez l'homme. D'autre part, le nombre restreint de patients enrôlés dans les essais cliniques ne permet pas de façon systématique l'identification de problèmes de toxicité associés à une population faible et spécifique. La mise au point, le développement et l'utilisation de tels tests permettraient d'identifier et d'éliminer le plus en amont possible les molécules toxiques. De nouveaux traitements pourraient ainsi être mis sur le marché plus précocement et à un coût moindre pour les entreprises pharmaceutiques et, par rebonds, les organismes de santé et les consommateurs. De plus, de tels tests pourraient également permettre de détecter certaines toxicités qui ne sont actuellement mises à jour qu'après mise sur le marché de composés.

Les tests actuellement disponibles ne permettent pas de suffisamment caractériser des marqueurs de toxicité et le potentiel de toxicité de molécules. Certains tests développés chez les bactéries (test de Ames) ou chez la levure (tel celui décrit par le brevet US 4,997,757) déterminent le pouvoir mutagène de composés. Ces tests ne peuvent détecter que des insultes faites au niveau de l'ADN. Or, de nombreuses molécules exercent des effets toxiques sans pour autant être mutagènes et ne peuvent donc pas être détectées par de tels outils. D'autres tests, tels celui décrit dans la demande WO 94/17208, utilisent un certain nombre de promoteurs de gènes eucaryotes connus ou d'éléments de réponse extraits de ces promoteurs, induits dans différentes situations de stress, afin de caractériser l'effet toxique de molécules. Toutefois, le nombre restreint de ces marqueurs génétiques et le processus mesuré (activité d'un promoteur) ne permettent pas de prédire le potentiel toxique de composés. En outre, ces tests sont difficiles à mettre en oeuvre puisqu'ils impliquent la culture de lignées cellulaires transformées, comprenant une ou plusieurs constructions génétiques.

WO 99/42606 concerne l'utilisation de certains poissons (teleostei et teleostomi) pour l'evaluation de la toxicité de composés.

La présente invention décrit maintenant des méthodes efficaces et rapides de détermination du potentiel toxique de composés tests, ainsi que tes outils et kits pour la mise en oeuvre de telles méthodes. Le terme « toxicité » désigne au sens de l'invention tout effet indésirable et/ou secondaire d'un composé sur le métabolisme d'une cellule ou d'un tissu, tel que notamment son potentiel mutagène, carcinogène, tératogène, etc., et plus généralement toute altération du métabolisme pouvant conduire à un effet néfaste du composé sur la cellule ou le tissu. La présente invention est plus particulièrement basée sur la génomique et la mise au point de marqueurs génétiques de la toxicité, utilisables pour prédire le caractère toxique de tout type de composé sur tout type de cellule. La présente invention décrit également de nouvelles méthodes pour obtenir des séquences d'acides nucléiques qui permettent de déterminer la toxicité de molécules (e.g., des marqueurs génétiques de la toxicité), en particulier de molécules qui entrent en développement pharmaceutique et /ou dans des compositions pharmaceutiques.

La présente invention repose en partie sur la mise en évidence que des marqueurs génétiques peuvent être créés et utilisés pour évaluer le caractère toxique de composés tests. En particulier, et de manière avantageuse, ces marqueurs sont utilisables quel que soit le composé toxique à tester, et quel que soit le type cellulaire sur lequel ce composé test est appliqué. De tels marqueurs, ainsi que les supports, kits et méthodes de l'invention permettent avantageusement de générer de manière rapide des profils de toxicité particulièrement évolués et fiables. En outre, ces marqueurs, supports, kits et méthodes de l'invention permettent aussi de déterminer et d'attribuer aux composés tests des index de toxicité.

En particulier, la présente invention montre qu'il existe des événements génétiques communs entre des situations de toxicité et des voies métaboliques cellulaires induites en l'absence de composés toxiques. De tels événements génétiques peuvent donc être induits, puis utilisés comme marqueurs de toxicité. Comme il sera décrit en détail dans la présente description, de tels marqueurs peuvent en outre être sélectionnés ou modifiés pour constituer des banques améliorées, permettant un diagnostic plus prédictif de la toxicité d'un composé. Plus spécifiquement, la présente invention montre maintenant que des marqueurs génétiques induits dans une cellule en situation de dérégulation de voie(s) de signalisation cellulaire, en particulier une cellule où la viabilité et/ou la prolifération cellulaire sont dérégulées (par exemple en situation d'apoptose) peuvent être utilisés pour caractériser de manière efficace le profil toxique de composés tests. De manière avantageuse, l'invention montre également que ces marqueurs peuvent être utilisés dans des tests de toxicité, indépendamment du type de composé et du type cellulaire utilisé. L'invention décrit également la constitution de banques différentielles d'acides nucléiques, caractéristiques de dérégulation(s) de voie(s) de signalisation cellulaire, notamment de situations dans lesquelles la viabilité et/ou la prolifération cellulaire sont dérégulées, et la mise en évidence que ces banques sont utilisables pour évaluer avec une fiabilité et une sensibilité importantes le profil toxique de composés.

L'invention permet également l'identification et/ou la caractérisation de mutations ou polymorphismes (en particulier de SNPs « Single Nucleotide Polymorphisms ») caractéristiques de profils de sujets, notamment du profil répondeur à un composé ou traitement, du profil sensible à un effet toxique, etc. Ainsi, les banques produites représentent une extraction de séquences qui sont mobilisées au cours de dérégulations de signalisation cellulaire et notamment lors de stress pouvant conduire à l'apoptose. L'invention montre que l'expression de ces séquences est modifiée de façon spécifique en réponse à des composés donnés. Des mutations ou polymorphismes, aussi désignés SNPs (single nucleotide polymorphisms), peuvent affecter les gènes dont dérivent ces séquences. Des individus qui présentent de tels polymorphismes sont donc susceptibles de réponses altérées aux composés mobilisant l'expression de ces gènes. L'un des aspects de l'invention est donc également de permettre une sélection pertinente des gènes impliqués dans les phénomènes toxiques. Plus précisément, l'invention au sein de cette sélection permet de réaliser une sélection supplémentaire des gènes impliqués dans la réponse à un composé donné. L'invention foumit une base pour la recherche de polymorphismes dans un nombre restreint (10 à 50) de gènes dont les modifications d'expression sont reliés à l'impact toxique d'un composé donné, celui-ci pouvant appartenir à toute classe pharmacologique et à toute famille chimique.

Un objet de l'invention réside donc plus particulièrement dans l'utilisation de marqueurs génétiques caractéristiques de situation(s) de dérégulation(s) de voie(s) de signalisation cellulaire (situation(s) de « dérégulation ») pour caractériser le profil toxique de composés tests. Plus préférentiellement, l'invention a pour objet l'utilisation de marqueurs génétiques induits en situation de dérégulation pour caractériser le profil toxique de composés tests. L'invention concerne plus préférentiellement l'utilisation de clones d'acides nucléiques correspondant à des événements génétiques (par exemple transcriptionnels et/ou d'épissage) caractéristiques de situation(s) de dérégulation comme marqueurs génétiques de toxicité.

L'invention a également pour objet des méthodes d'analyse du potentiel toxique d'un composé test, comprenant au moins une étape d'hybridation entre a) un échantillon d'acides nucléiques de cellules traitées par ce composé et b) une préparation (par exemple une banque) d'acides nucléiques correspondant à des événements génétiques caractéristiques de situation(s) de dérégulation, le profil d'hybridation indiquant le potentiel toxique du composé test.

L'invention réside encore dans des kits pour la mise en oeuvre de ces méthodes, ainsi que dans des compositions ou banques d'acides nucléiques comprenant des marqueurs génétiques de dérégulation(s) des voies de signalisation cellulaire, ou également dans des méthodes permettant de générer de tels marqueurs.

L'invention concerne également l'utilisation des clones ou acides nucléiques décrits, pour l'identification de mutations ou polymorphismes, notamment de SNPs, correlés à la réponse de sujets à des composés pharmaceutiques. L'invention permet en effet d'identifier un ensemble de gènes dont les expressions sont modifiées en réponse à un composé donné. Ces gènes peuvent présenter au sein des populations humaines des mutations ou polymorphismes (dont les SNPs, single nucleotide polymorphisms). L'invention permet de sélectionner les gènes et leurs polymorphismes à étudier afin de ségréguer des patients en fonction de leur génotype pour leur capacité à répondre de façon plus ou moins optimale à un composé et plus particulièrement en minimisant les risques de toxicité.

A cet égard, l'invention concerne, plus généralement, l'utilisation d'acides nucléiques représentatifs d'événements d'épissages caractéristiques d'une situation physiopathologique donnée, pour l'identification ou la caractérisation de SNPs correlés à ladite situation.

L'invention concerne également une méthode d'identification de SNPs ou autres mutations ou polymorphismes permettant d'évaluer la réponse d'un sujet à un composé donné, comprenant (i) l'identification in vitro d'acides nucléiques caractéristiques d'événements d'épissages induits dans une cellule traitée par ledit composé et (ii) l'identification de SNPs ou autres mutations ou polymorphismes dans le ou les gènes correspondant à des acides nucléiques identifiés en (i). L'invention fournit ainsi des méthodes et moyens pour sélectionner des gènes pour lesquels des polymorphismes sont associés à une toxicité pour un composé donné.

L'invention concerne également une population d'acides nucléiques spécifiques de polymorphismes ou SNPs ainsi identifiés, notamment d'amorces nucléotidiques pour l'amplification sélective des polymorphismes, ou de sondes pour l'hybridation sélective avec les polymorphismes considérés.

L'invention concerne également des méthodes pour l'analyse (par exemple la prédiction, l'évaluation ou la détermination) de la réponse d'un sujet à un composé test, comprenant l'étude de polymorphismes, notamment l'analyse de SNPs, tels que définis ci-avant.

La présente invention repose donc notamment sur la définition et la mise au point de marqueurs génétiques de la toxicité, utilisables dans des méthodes de prédiction du caractère toxique de composés test, ou comme cible pour l'étude des mécanismes de mort cellulaire et la recherche ou le développement de produits thérapeutiques.

### 1. Définition et mise au point de marqueurs génétiques de la toxicité

La viabilité et la différentiation cellulaires sont régulées par la balance qui existe entre les phénomènes de mitose et d'apoptose. Dans l'organisme, l'homéostasie tissulaire est également réglée par cette balance entre prolifération et mort cellulaires. Les altérations de cet équilibre constituent la base des pathologies, qu'elles soient liées à un excès (maladies neurodégénératives) ou à un défaut d'apoptose (polyarthrite rhumatoïde, cancers). La présente invention est fondée notamment sur l'hypothèse que les perturbations de cet équilibre peuvent également être impliquées dans des phénomènes toxiques et que des marqueurs génétiques caractéristiques de ces événements de dérégulation des voies de signalisation cellulaire (notamment de la viabilité et/ou de la prolifération cellulaire) pourraient constituer des marqueurs efficaces de la toxicité.

La présente invention montre maintenant qu'il existe des événements génétiques communs entre ces situations de dérégulation des voies de signalisations cellulaires et des situations de toxicité induite par des composés. La présente invention fournit également des méthodes pour identifier, caractériser, sélectionner et isoler des clones d'acides nucléiques correspondant à ces événements génétiques, et montre qu'ils peuvent être utilisés efficacement comme marqueurs génétiques de la toxicité. L'invention décrit également la constitution de banques de tels clones, notamment sur supports solides, et leur utilisation dans des méthodes de diagnostic de la toxicité de composés test.

### 2. Définitions

Dans le contexte de la présente invention, le terme situation de dérégulation ou « dérégulation » désigne toute situation de dérégulation ou altération d'une ou plusieurs voies de signalisation cellulaire, et en particulier toute situation de dérégulation de la croissance et/ou de la viabilité cellulaire. Il s'agit donc de toute cellule dans laquelle une ou plusieurs voies de signalisation cellulaires ont été altérées (c'est-à-dire notamment induite ou réprimée), par exemple une situation d'apoptose, comme il sera décrit plus en détails dans la suite du texte.

Dans le contexte de la présente invention, l'expression " événement génétique caractéristique d'une situation de dérégulation" désigne plus particulièrement toute modification de l'activité génétique, notamment de l'expression de gènes (augmentation ou répression, suppression ou induction, etc .), de la régulation post-transcriptionnelle (notamment de l'épissage), de la réplication, l'apparition de délétions, etc., caractéristique d'une dérégulation, c'est-à-dire induite dans une cellule en situation de dérégulation. Il est entendu que chaque événement génétique individuel caractéristique de dérégulation n'est pas nécessairement spécifique de cette situation, dans la mesure où certaines voies de signalisation cellulaire participent à des processus cellulaires multiples. Cependant, les banques d'acides nucléiques de l'invention regroupent différents clones et représentent donc effectivement des signatures génétiques caractéristiques de telles situations.
Les clones de l'invention correspondent plus préférentiellement à des événements génétiques transcriptionnels et/ou d'épissage caractéristiques de dérégulation(s). Dans le contexte de l'invention, le terme " événements transcriptionnels " englobe plus particulièrement toute activité d'expression de gènes, c'est-à-dire la production de transcrits primaires, pré-messagers ou messagers à partir de régions codantes. L'expression " événements d'épissages " désigne plus particulièrement tout événement d'épissage lié à une situation de dérégulation, c'est-à-dire l'apparition de formes particulières d'épissage, la disparition de formes particulières d'épissage, ou éventuellement la modulation de la quantité de formes d'épissage, etc.

L'invention décrit donc la production de clones d'acides nucléiques correspondant à de tels événements génétiques caractéristiques de situations où une ou plusieurs voies de signalisation cellulaire sont altérées, et leur utilisation comme marqueurs génétiques de la toxicité. Un clone d'acide nucléique correspondant à un tel événement génétique désigne au sens de l'invention tout acide nucléique ou fragment d'acide nucléique comprenant au moins une région dont la séquence est spécifique de cet événement. Ainsi, il peut s'agir de tout acide nucléique comprenant une séquence spécifique d'une forme d'épissage ou d'un délétant caractéristique d'une situation de dérégulation ou encore d'un gène exprimé ou régulé spécifiquement en situation de dérégulation. De tels clones sont donc des acides nucléiques susceptibles d'hybrider, au sein d'un échantillon d'acide nucléique test, avec les formes d'épissage caractéristiques de la situation de dérégulation ou avec les gènes exprimés préférentiellement ou spécifiquement lors de la situation de dérégulation. Ces clones permettent ainsi de révéler, par hybridation, la présence des événements génétiques ciblés dans, une population d'acides nucléiques test.

### 3. Préparation des marqueurs génétiques de la toxicité

Comme indiqué ci avant, la présente invention montre que des marqueurs génétiques caractéristiques de situations de dérégulation peuvent être utilisés comme marqueurs de la toxicité, et décrit à cet égard la mise au point de techniques permettant de générer et d'isoler de tels marqueurs.

Un objet de l'invention réside donc dans des méthodes permettant de générer des marqueurs génétiques de la toxicité. Les méthodes de l'invention comprennent plus particulièrement la constitution de clones et de banques d'acides nucléiques caractéristiques de dérégulation(s) de voie(s) de signalisation cellulaire(s). Ces clones et banques peuvent être générés de différentes manières, comme il sera décrit en détails dans ce qui suit. Par ailleurs, les banques de l'invention peuvent comporter des quantités variables de clones d'acides nucléiques. En outre, ces banques sont avantageusement déposées sur des supports, facilitant l'étape d'hybridation et d'analyse du profil d'hybridation.

Le matériel de départ utilisé pour la production des clones et banques comprend essentiellement des populations d'acides nucléiques dérivées de cellules en situation de dérégulation, et des populations d'acides nucléiques dérivées de cellules en situation de contrôle. Dans un mode plus particulier de mise en oeuvre, le procédé de production de marqueurs génétiques de la toxicité selon l'invention comprend avantageusement une étape d'hybridation entre un échantillon d'acides nucléiques dérivé d'une cellule en situation de dérégulation et un échantillon d'acides nucléiques dérivé d'une cellule en situation de contrôle. Cette étape d'hybridation permet de générer des (banques de) clones d'acides nucléiques caractéristiques de la cellule en situation de dérégulation par rapport à la situation contrôle.

Les populations d'acides nucléiques utilisées sont par exemple des ARN totaux ou des ARN messagers des cellules en situation de dérégulation et de contrôle, ou des acides nucléiques dérivés des ces ARN totaux ou messagers (par transcription inverse, amplification, clonage dans des vecteurs, etc.). Ces populations peuvent être préparées par des techniques conventionnelles connues de l'homme du métier (transcription inverse, amplification, etc.).

Le choix de la ou des situations de dérégulation, de la ou des situations de contrôle, ainsi que des techniques de production et d'isolement des marqueurs sont importants puisqu'ils déterminent la pertinence des marqueurs générés, et donc des banques, ainsi que pour leur facilité d'utilisation.

### 3.1. Matériel de départ

Comme indiqué ci avant, les marqueurs génétiques de la toxicité selon l'invention sont généralement préparés initialement à partir de préparations d'acides nucléiques provenant de cellules en situation de dérégulation. Il est entendu que ces marqueurs, une fois isolés et caractérisés, peuvent être reproduits ou amplifiés par des méthodes artificielles, telles que la PCR, la synthèse artificielle (lorsque les séquences sont disponibles), l'amplification par culture bactérienne, la réplication de banques, etc., comme il sera illustré plus loin.

Comme indiqué ci-avant, on entend, dans le cadre de la présente invention, par " situation de dérégulation", toute situation de dérégulation de la croissance et/ou de la viabilité cellulaire, par exemple toute cellule dans laquelle une voie de signalisation cellulaire au moins a été altérée.

Selon une variante particulière de l'invention, la cellule en situation de dérégulation est une cellule dans laquelle l'expression de tout ou partie d'un gène impliqué dans la croissance ou la viabilité cellulaire a été modifiée.

Selon une autre variante particulière de l'invention, la cellule en situation de dérégulation est une cellule dans laquelle l'expression de tout ou partie d'un oncogène ou d'un anti-oncogène impliqué a été modifiée.

Selon une autre variante particulière de l'invention, la cellule en situation de dérégulation est une cellule dans laquelle l'expression de tout ou partie d'un gène impliqué dans l'apoptose cellulaire a été modifiée.

L'apoptose cellulaire est un terme connu de l'homme du métier, désignant le . processus de mort cellulaire programmé. L'apoptose englobe un ensemble de mécanismes, de gènes ou de voies métaboliques cellulaires qui conduisent à la mort de la cellule. Certains des systèmes initiateurs et exécuteurs de l'apoptose, sont par exemple les anti-oncogènes, les récepteurs Fas et au TNF, des adaptateurs (à " death domain " notamment), des membres de la famille bcl2 ou encore des caspases, qui sont impliqués dans la morphogenèse ontogénique, dans les dérégulations pathologiques et lors de la mort cellulaire.

Dans le cadre de l'invention, une situation d'apoptose peut être toute situation d'initiation, d'exécution ou de terminaison de l'apoptose, c'est à dire une situation d'apoptose à tout stade d'avancement du processus de mort cellulaire, y compris des stades très précoces dans lesquels certaines voies métaboliques (ou voies de signalisation cellulaires) seulement ont été altérées (i.e., activées, réprimées, stimulées, etc.). Les clones de l'invention peuvent donc être produits à partir de toute cellule dans laquelle au moins une voie de signalisation cellulaire a été altérée, en particulier dans laquelle une voie apoptotique a été au moins initiée. Il peut ainsi s'agir de cellules dans lesquelles une voie métabolique participant au processus d'apoptose a été induite ou stimulée, même si cette induction ou stimulation est réalisée dans des conditions sub-létales (i.e., ne conduisant pas à la mort de la cellule).
A cet égard, il peut s'agir de cellules en situation naturelle d'apoptose (comme par exemple une population de cellules dans des tumeurs, certaines cellules nerveuses dans des situations pathologiques, etc.), ou bien de cellules dans lesquelles la situation d'apoptose a été artificiellement induite. Dans ce dernier cas, la situation d'apoptose peut être induite par différents types de traitements, et notamment par la modulation de l'activité, préférentiellement de l'expression de gènes initiateurs ou exécuteurs de l'apoptose.

Dans un mode particulier, les marqueurs génétiques de l'invention sont produits initialement à partir d'une population de cellules dans laquelle une situation d'apoptose a été induite artificiellement, par induction ou répression de l'activité, préférentiellement de l'expression de tout ou partie d'un gène initiateur ou exécuteur de l'apoptose.

Selon une variante préférée de l'invention, la cellule en situation de dérégulation est une cellule dans laquelle l'activation, préférentiellement l'expression de tout ou partie d'un gène anti-oncogène (ou gène suppresseur de tumeur) a été induite ou augmentée, par exemple par surexpression, modification(s) post-traductionnelle(s) (phosphorylation, glycosylation, etc.), localisation intracellulaire dérégulée, association avec des partenaires fonctionnels différents, etc.

Dans un mode particulièrement préféré de mise en oeuvre de la présente invention, les événements génétiques caractéristiques d'une situation de dérégulation sont les événements génétiques induits par modification de l'expression d'un ou plusieurs gènes initiateurs ou exécuteurs de l'apoptose, notamment d'un ou plusieurs gènes suppresseurs de tumeurs (ou d'un variant fonctionnel de tels gènes).

Il peut s'agir notamment de toute cellule dans laquelle une surexpression d'un anti-oncogène a été induite. En particulier, il peut s'agir d'une cellule dans laquelle une construction génétique a été introduite, permettant d'induire ou d'augmenter la quantité d'anti-oncogène dans cette cellule. Des exemples particuliers de gènes anti-oncogènes (également désignés gènes suppresseurs de tumeurs) sont notamment p53 (Eliyahu et al., Proc Natl Acad Sci U S A (1989) 86:8763-7 ; Finlay et al., Cell (1989) 57,1083-93), Rb (Lee et al., Science (1987) 235,1394-9), p73 (Kaghad et al., Cell (1997) 90, 809-19), TUPRO-2 (US5,849,899), NHTS (US5,892,016), p15 (Hannon et al., Nature 371 (1994) 257), p16 (Ivison et al., Nature Genet. 371 (1994) 180), etc., mais ne sont pas limités à ces seuls exemples.

Un exemple typique de situation de dérégulation selon l'invention est généré par induction ou augmentation de l'activité, préférentiellement de l'expression de p53. Au carrefour entre vie et mort cellulaire, se situe p53, gène suppresseur de tumeur (ou anti-oncogène), qui régule négativement la croissance cellulaire en provoquant un arrêt en fin de phase G1. Cet arrêt du cycle cellulaire est caractérisé par une répression de l'expression des gènes impliqués dans la progression de la phase G1 à la phase S et l'induction de gènes spécifiques. L'abolition de ces fonctions de p53 est l'un des mécanismes en jeu dans la formation d'un grand nombre de tumeurs où la délétion ou des mutations dans le gène p53 sont observées. Une surexpression de p53 peut induire l'apoptose dans différents types cellulaires. Cette surexpression induite par infection à l'aide de virus qui permettent l'expression de la forme sauvage de p53 est la base de traitements de cancers par thérapie génique. L'induction de l'expression de p53 peut également être observée en physiologie et a notamment été décrite lors de la restructuration de la glande mammaire liée à l'arrêt de la lactation. Une induction d'apoptose par retrait de sérum, situation qui affecte un grand nombre de voies métaboliques, a également été décrite impliquant une stabilisation de p53.

L'induction de p53 engage, dans les cellules, un programme de mort cellulaire lorsque les cassures de l'ADN débordent les systèmes de réparation de la cellule. Cette induction d'apoptose met à contribution notamment le système Fas et les caspases dont les messagers doivent être épissés de façon à ce que soient exprimées les isoformes compétentes pour la mort cellulaire.

En outre, p53 est connu pour réguler l'expression de gènes impliqués dans d'autres processus cellulaires variés comme la régulation du cycle cellulaire, l'angiogénèse, la réponse au stress oxydatif, l'altération de l'ADN ou la croissance cellulaire. P53 exerce par ailleurs un rôle direct sur la réponse cellulaires aux altération de l'ADN, l'hypoxie, le potentiel redox des cellules, ou l'adhésion cellulaire. Par ailleurs, il a été montré que p53 pouvait migrer depuis le noyau cellulaire jusqu'aux mitochondries, où elle est capable d'interagir avec la protéine hsp70, ce ciblage mitochondrial pouvant causer la mort cellulaire. Les signatures obtenues selon la présente invention après induction de p53 permettent donc d'étudier l'ensemble de ces voies de signalisation et de fournir une réponse complète et prédictive du potentiel toxique de composés.

L'invention décrit plus particulièrement l'utilisation de marqueurs génétiques induits par l'activation, préférentiellement l'expression de p53 (en particulier p53 sauvage) pour caractériser le profil toxique de composés tests. L'invention concerne plus préférentiellement l'utilisation de clones d'acides nucléiques correspondant à des événements génétiques (par exemple transcriptionnels et/ou d'épissage) induits par l'activation, préférentiellement l'expression de p53 (en particulier p53 sauvage) comme marqueurs génétiques de toxicité.
L'invention décrit plus particulièrement l'obtention et l'utilisation de clones d'ADNc qui correspondent à des événements génétiques qualitatifs et /ou quantitatifs différentiels entre des cellules contrôles (par exemple en prolifération) et des cellules engagées dans un processus d'apoptose par induction de la forme sauvage de p53.

Il est entendu que d'autres possibilités existent de soumettre des cellules à un stress susceptible de les engager sur la voie de la mort cellulaire programmée (apoptose) ou d'altérer des voies de signalisation cellulaire. L'invention concerne donc également l'utilisation d'autres modèles cellulaires tels que ceux basés sur l'expression inductible (ou constitutive) de tout ou partie de gènes d'anti-oncogènes, de promoteurs, initiateurs et exécuteurs d'apoptose.

Parmi ces gènes peuvent être choisis avantageusement RB, le produit du gène du rétinoblastome, p73, protéine homologue à p53, myc ou toute autre protéine ou fragment de protéine capable d'interférer avec la croissance et la viabilité cellulaire. Comme indiqué ci avant, il peut également s'agir par exemple d'une cellule tumorale ou d'une cellule nerveuse en dégénérescence.

D'autres types de matériel de départ sont constitués par exemple d'échantillons contenant des cellules dans lesquelles la prolifération est dérégulée par activation de cascades oncogéniques (telles que la voie de signalisation ras), inactivation de gènes suppresseurs de tumeurs, inhibition de cascades apoptotiques, inhibition ou activation de voies médiées par des kinases (par exemple la kinase pl3, qui stimule la protéine Akt), inhibition de l'expression de gènes au moyen d'ARN ou d'oligonucléotides antisens, etc. Un exemple particulier de cellules est fourni par des cellules tumorales obtenues à partir de biopsies de tumeurs (dans ce cas, les marqueurs génétiques selon l'invention sont ceux caractéristiques de la cellule tumorale par rapport à un échantillon de tissu contrôle, notamment sain, prélevé par biopsie).

En outre, la situation de dérégulation peut également être induite par un ou plusieurs composés toxiques sélectionnés, agissant sur la viabilité cellulaire selon des processus qui affectent l'intégrité du génome (génotoxicité), le potentiel redox de la cellule, la réponse initiée par certains récepteurs de surface, la conformation des protéines ou le statut énergétique de la cellule par exemple. De tels composés peuvent en particulier être utilisés en combinaison avec les approches d'induction décrites précédemment, basées sur des modifications de l'expression de gènes.

La population cellulaire utilisée pour induire une situation de dérégulation peut être d'origine et de nature diverses, telles que des cellules épithéliales, hépatiques, pulmonaires, nerveuses, musculaire, fibroblastiques, etc. Il s'agit de manière préférentielle de cellules humaines. Il peut s'agir de cultures primaires ou de lignées établies, dans lesquelles la dérégulation est induite dans les conditions décrites ci-avant. Il peut également s'agir d'échantillons tumoraux qui comporteront des cellules en situation de dérégulation ou de cellules privées de sérum par exemple. Comme il est indiqué plus loin, des marqueurs génétiques de dérégulation utilisables comme marqueurs de la toxicité selon l'invention peuvent être produits à partir de différents types de cellules.

La situation de contrôle utilisée est généralement une population de cellules du même type que la cellule utilisée pour la situation de dérégulation, même si des types cellulaires différents peuvent être utilisés. La situation de contrôle peut être une situation normale quiescente, proliférative ou même de dérégulation, mais à un degré ou un stade moins avancé que la situation de dérégulation de référence.

Typiquement, la situation de dérégulation est une situation d'induction de l'activation, préférentiellement l'expression d'un gène suppresseur de tumeur, tel p53 ou Rb, et la situation de contrôle est une situation d'absence d'induction de cette activation, préférentiellement expression, ou d'induction à un niveau plus faible. Dans un autre exemple, la situation de dérégulation est une population de cellules cancéreuses et la situation contrôle est une population des mêmes cellules à l'état sain. Des exemples spécifiques de cellules sont données dans la section expérimentale.

### 3.2. Production des marqueurs et des banques

Comme indiqué ci avant, le procédé de production de marqueurs génétiques de la toxicité selon la présente invention comprend avantageusement une étape d'hybridation entre un échantillon d'acides nucléiques dérivé d'une cellule en situation de dérégulation et un échantillon d'acides nucléiques dérivé d'une cellule en situation de contrôle. Cette étape d'hybridation permet de générer des clones d'acides nucléiques caractéristiques de la cellule en situation de dérégulation par rapport à la situation contrôle.

Les populations d'acides nucléiques sont par exemple des ARN totaux ou des ARN messagers des cellules en situation de dérégulation et de contrôle, ou des acides nucléiques dérivés des ces ARN totaux ou messagers (par transcription inverse, amplification, clonage dans des vecteurs, etc.). Ces acides nucléiques peuvent être préparés selon les techniques connues de l'homme du métier. Brièvement, ces techniques comprennent généralement une lyse des cellules, tissu ou échantillon, et l'isolement des ARNs par des techniques d'extraction. Il peut s'agir en particulier d'un traitement au moyen d'agents chaotropiques tels que le thiocyanate de guanidium (qui détruit les cellules et protège les ARN) suivi d'une extraction des ARN au moyen de solvants (phénol, chloroforme par exemple). De telles méthodes sont bien connues de l'homme du métier (voir Maniatis et al., Chomczynski et al., Anal. Biochem. 162 (1987) 156), et peuvent être aisément pratiquées en utilisant des kits disponibles dans le commerce tels que par exemple le kit US73750 (Amersham) pour les ARN totaux. Il n'est pas nécessaire que les ARN utilisés soient parfaitement purs, et notamment il n'est pas gênant que des traces d'ADN génomique ou d'autres composants cellulaires (protéine, etc.) subsistent dans les préparations, dès lors qu'ils n'affectent pas significativement la stabilité des ARNs. En outre, de manière facultative, il est possible d'utiliser non pas des préparations d'ARN totaux mais des préparations d'ARN messagers. Ceux-ci peuvent être isolés, soit directement à partir de l'échantillon biologique soit à partir des ARN totaux, au moyen de séquences polyT, selon les méthodes classiques. L'obtention d'ARN messagers peut à cet égard être réalisée au moyen de kits commerciaux tels que par exemple le kit US72700 (Amersham). Les ARN peuvent également être obtenus directement à partir de banques ou autres échantillons préparés à l'avance et/ou accessibles dans des collections, conservés dans des conditions appropriées.

L'hybridation peut être réalisée dans différentes conditions, qui peuvent être ajustées par l'homme du métier. De préférence, on utilise pour l'hybridation un excès de la population d'acides nucléiques dérivés de la situation de dérégulation par rapport à la population d'acides nucléiques dérivés de la situation de contrôle.

A partir du produit de la réaction d'hybridation, deux types principaux d'approches peuvent être utilisés pour isoler les clones caractéristiques de dérégulation selon l'invention. La première, purement quantitative, permet de générer une préparation d'acides nucléiques regroupant l'ensemble (ou une part significative) des clones résultant d'une différence de niveau d'expression entre les deux situations. De tels clones (et banques) sont obtenus selon les techniques connues d'hybridation soustractive, consistant essentiellement à éliminer les hybrides formés lors de l'étape d'hybridation, pour ne conserver que les clones non hybridés, caractéristiques de la situation de dérégulation par rapport à la situation de contrôle choisie.

Dans un mode préféré de mise en oeuvre, on utilise cependant un procédé qualitatif, qui permet de générer une préparation d'acides nucléiques regroupant l'ensemble (ou une partie importante) des clones résultant d'altérations génétiques fonctionnelles caractéristiques de la situation de dérégulation par rapport à la situation de contrôle choisie. Plus particulièrement, une telle banque qualitative comprend non pas l'ensemble des clones dont l'expression est modifiée, mais par exemple les clones correspondant à des épissages ou à des délétions différentiels entre les deux situations. Compte tenu du rôle des épissages alternatifs dans les voies de régulation et de transformation cellulaires, de telles préparations (et banques) comportent avantageusement des clones ayant une valeur fonctionnelle importante, et donc susceptibles de refléter des modifications génétiques impliquées dans la situation de dérégulation. De tels clones permettent donc de constituer des banques plus prédictives et de générer des marqueurs génétiques plus représentatifs.
La constitution de telles banques qualitatives peut être réalisée par isolement, à partir des hybrides formés lors de l'étape d'hybridation, des régions d'acides nucléiques correspondant à des épissages différentiels ou à des délétions. Selon les méthodes employées, ces régions correspondent soit aux régions non appariées, soit aux régions appariées.

Ces deux approches sont décrites plus en détails dans ce qui suit.

### 3.2.1. Production et utilisation de banques différentielles quantitatives

Dans un premier mode de mise en oeuvre, on utilise donc dans la présente invention une banque différentielle quantitative, c'est à dire une banque comprenant des clones d'acides nucléiques correspondant à des gènes dont le niveau d'expression est modifié dans des cellules en situation de dérégulation par rapport à une situation contrôle. De telles banques peuvent être issues par exemple d'analyses différentielles quantitatives, et regrouper les séquences dont l'expression est augmentée ou diminuée lors de phénomènes de dérégulation cellulaire. Les méthodologies pour établir ce type de banque sont connues de l'homme de métier et peuvent être regroupées dans les catégories suivantes :

### Soustraction électronique à partir de Séquençage à Haut Flux

Ce procédé est basé sur le séquençage aléatoire d'un certain nombre de cDNAs. Un moteur de recherche informatique peut ensuite être utilisé pour effectuer une soustraction entre deux situations analysées.

### « Sérial Analysis of Gene Expression (SAGE) »

Ce procédé est basé sur la reconnaissance d'une signature associée à chaque cDNA en utilisant des enzymes de restriction et des oligonucléotides adaptateurs. Cette étiquette correspond à une partie de la séquence du cDNA (longue de 10 nucléotides afin d'identifier de façon non ambiguë le cDNA correspondant). Ces étiquettes sont ensuite assemblées pour être séquencées puis analysées (Velculescu et coll., Science, 1995, 270 :484-487). Cette approche représente donc un raccourci vis-à-vis du séquençage systématique.

### « Nucleic Acid Arrays »

Cette méthode repose sur le dépôt d'acides nucléiques (oligonucléotides, fragments PCR, cDNAs) sur supports solides (membranes, plaques de verre, bio-puces) à plus ou moins haute densité. Des sondes provenant d'ARN messagers d'échantillons sains ou pathologiques sont ensuite utilisées pour hybridation afin d' identifier les messagers qui sont ou bien sur-exprimés ou bien réprimés.

### « Differential Display »

Cette technique utilise une amorce oligo-dT et des amorces aléatoires pour réaliser des réactions PCR sur des populations d' ADNc. Les produits de PCR sont alors comparés sur des gels très résolutifs. Les fragments exprimés de façon différentielle sont ensuite isolés et leur présence est confirmée par Northem-blots avant séquençage.
Plusieurs variantes de cette technologie ont été développées (Prashar et Weissman, PNAS, 1996, 93:659-663). Ces variantes diffèrent par leur amorces et par le choix des enzymes de restriction et des adaptateurs utilisés. Comme la technologie SAGE, elles s'adressent aux extrémités 3' des cDNAs. Plusieurs kits sont également disponibles sur le marché afin de rendre accessible cette approche.

### Clonage par soustraction

Cette technique est basée sur l'élimination de cDNAs communs à deux échantillons que l'on désire comparer. Ainsi, différents kits de soustraction dans lesquels le cDNA 'tester' est hybridé à un excès de cDNA 'driver' sont proposés (Clontech). Le produit final est constitué d'un pool de fragments amplifiés par PCR dérivé des cDNAs exprimés de façon différentielle, qui peut être cloné dans un vecteur approprié pour analyse ultérieure. La technologie RDA (Representational Difference Analysis) est également basée sur ce principe de soustraction (Lisitsyn et coll., Science, 1993, 259 :946-951.

La mise en oeuvre de ces techniques d'analyses différentielles permet donc de générer des clones et des banques quantitatifs, c'est-à-dire regroupant l'ensemble des séquences dont l'expression est augmentée ou diminuée lors de phénomènes de dérégulation(s) cellulaire(s).

### 3.2.2. Production et utilisation de banques qualitatives différentielles

Dans un autre mode de mise en oeuvre, on utilise avantageusement dans la présente invention une banque différentielle qualitative, c'est à dire une banque comprenant des clones d'acides nucléiques dont une partie au moins de la séquence correspond à la séquence de gènes épissés différentiellement dans des cellules en situation de dérégulation. Ce type de banque regroupe donc les séquences épissées de manière différentielle lors de processus de dérégulation.

L'utilisation de ce type de banque est particulièrement avantageuse. En effet, différents stress cellulaires qui conduisent à la mort cellulaire ou à d'autres dérégulations mettent en oeuvre des initiateurs et des exécuteurs des voies de signalisation et régulent les éléments clefs du cycle cellulaire, dont p53. La régulation du niveau d'expression de cette protéine se fait principalement par sa stabilisation et par augmentation transcriptionnelle. Si les récepteur Fas, les membres de la famille bcl2 et les caspases sont régulés au niveau de leur transcription, ils sont de plus contrôlés au niveau de la maturation, de l'épissage, de leur ARN pré-messager. Ce niveau de régulation post transcriptionnel est critique puisqu'il détermine, à partir d'un même événement de transcription, la genèse d'une forme pro- ou anti-apoptotique de chacun des membres des familles protéiques concernées. Ces modifications de la transcription et de l'épissage sont régulées par le métabolisme et la signalisation cellulaires. Les facteurs de régulation de la transcription et de l'épissage qui régulent en "trans" ces étapes clefs de l'expression génique sont des protéines dont l'activité est régulée, par phosphorylation notamment, selon l'état de prolifération, de différentiation ou de viabilité de la cellule. Le programme d'épissage qui est engagé lors de dérégulations cellulaires et qui mobilise les différents initiateurs et exécuteurs de la mort cellulaire traduit donc les modifications de signalisation cellulaire dues aux différentes agressions. En affectant les machineries de la transcription et de l'épissage, ces modifications jouent sur l'expression de nombreux gènes qui ne sont pas limités aux gènes décrits pour jouer un rôle d'initiateurs ou d'exécuteurs de dérégulation mais interviennent à différents niveaux des cascades de signalisation. De même, les cascades altérées lors de l'oncogénèse, notamment par expression de variants d'épissages, mobilise des marqueurs dont l'expression n'est pas restreinte aux tumeurs.

Pour tenir compte de ces phénomènes et de cette complexité, et foumir ainsi une réponse plus prédictive sur le potentiel toxique d'un composé test, le procédé de l'invention utilise donc avantageusement comme marqueurs génétiques de la toxicité, des événements d'épissages caractéristiques de situations de dérégulation.

Pour ce faire, la présente invention utilise par exemple des banques d'acides nucléiques qualitatives différentielles produites selon la méthodologie « DATAS » décrite dans la demande de brevet internationale n° WO99/46403. En particulier, de telles banques peuvent être préparées par hybridation entre la population d'acides nucléiques dérivée des cellules en situation de dérégulation et la population d'acides nucléiques dérivée des cellule en situation de contrôle, et isolement, à partir des hybrides formés, des acides nucléiques correspondant à des épissages différentiels.

Dans cette approche, l'hybridation est préférentiellement réalisée en phase liquide. En outre, elle peut être effectuée dans tout dispositif approprié, tel que par exemple des tubes (Eppendorff, par exemple), des plaques, ou tout autre support adapté et couramment utilisé en Biologie Moléculaire. L'hybridation est avantageusement réalisée dans des volumes compris entre 10 et 1000 µl, par exemple entre 10 et 500 µl. Il est entendu que le dispositif utilisé et les volumes utilisés peuvent être aisément adaptés par l'homme du métier. Les quantités d'acides nucléiques utilisées pour l'hybridation sont également connues de l'homme du métier. En général, il est suffisant d'utiliser des microgrammes d'acides nucléiques, par exemple de l'ordre de 0,1 à 100 µg. Par ailleurs, il est possible d'utiliser les acides nucléiques dans un rapport driver/tester variant de 50 à 0,02 environ, de préférence de 40 à 0,1. De manière plus particulièrement avantageuse, on préfère que ce rapport soit proche ou supérieur à 1, avantageusement entre 1 environ et 10 environ. Il est bien entendu que ce rapport peut être adapté par l'homme du métier selon les conditions du procédé (quantités d'acides nucléiques disponibles, situations physiologiques, but poursuivi, etc.). Les autres paramètres de l'hybridation (temps, température, force ionique) sont également adaptables par l'homme du métier. De manière générale après dénaturation des "tester" et "driver" (par chauffage par exemple), l'hybridation est réalisée pendant environ 2 à 24 heures, à une température de 37°C environ (éventuellement soumise à des sauts de température), et dans des conditions standard de force ionique (pouvant varier de 0,1 à 5M NaCl par exemple). Il est connu que la force ionique est un des facteurs déterminant la stringence d'une hybridation, notamment dans le cas d'hybridation sur support solide.
Selon un mode de mise en oeuvre particulier de l'invention, l'hybridation est réalisée en émulsion phénolique, par exemple selon la technique PERT ("Phenol Emulsion DNA Reassociation Technique) décrite par Kohne D.E. et al. (Biochemistry, Vol. 16, N° 24, pp 5329-5341, 1977). Avantageusement, l'hybridation est réalisée en émulsion phénolique maintenue par thermocycles (sauts de température de 37°C environ à 60/65°C environ) et non par agitation, selon la technique décrite par Miller et Riblet (NAR 23 (1995) 2339).

Toute autre technique d'hybridation en phase liquide, de préférence en émulsion, peut être utilisée dans le cadre de la présente invention. Par ailleurs, l'hybridation peut également se faire avec l'un des partenaires immobilisé sur un support. Avantageusement, c'est le driver qui est immobilisé. Cela peut être réalisé notamment grâce à des amorces biotinylées ou par toute autre technique d'immobilisation connue de l'homme du métier.

A partir des populations d'acides nucléiques générées par hybridation, les marqueurs génétiques de l'invention (les clones caractéristiques des altérations génomiques qualitatives) peuvent être identifiés par toute technique connue de l'homme du métier. Dans le cas des hétéroduplex ARN/ADN, ces régions se présentent essentiellement sous forme de régions d'ARN non-appariées (boudes d'ARN), et peuvent être identifiées et clonées par séparation des hétéroduplex et des acides nucléiques simple-brin (excès d'acide nucléique n'ayant pas réagi), digestion sélective des ARN double-brins (domaines engagés dans les hétéroduplex), puis séparation des ARN simple-brin résultant et des ADN simple-brins. Dans le cas des hétérotriplex, ces régions d'épissages différentiels se présentent essentiellement sous forme de régions d'ADN double-brin et peuvent être identifiées et donées par traitement en présence d'enzymes appropriées telles qu'une enzyme permettant de digérer les ARN, puis une enzyme permettant de digérer les ADN simple-brin. Les acides nucléiques ainsi obtenus sont directement sous forme d'ADN double-brin et peut être donés dans tout vecteur approprié.

Il est entendu que d'autres variantes et conditions précises pour l'isolement des acides nucléiques, les hybridations et l'obtention des clones qualitatifs sont indiquées dans la demande n° WO99/46403.

Ces méthodologies permettent de générer des clones et des banques d'acides nucléiques correspondant à des marqueurs génétiques qualitatifs de situation(s) de dérégulation. Comme indiqué dans la section expérimentale, ces préparations d'acides nucléiques représentent des marqueurs particulièrement utiles pour caractériser le profil toxique de composés.

### 4. Diversité des banques

Les méthodologies décrites ci avant permettent donc de générer des ensembles de clones d'acides nucléiques caractéristiques de situations de dérégulation. Chaque technique de préparation génère de nombreux clones, constituant des banques. Ces banques peuvent être mises en oeuvre telles quelles, déposées sur des supports, ou modifiées par addition ou suppression de clones, groupage entre différentes banques, ajout de clones témoins, etc.

Les banques de l'invention peuvent comprendre par exemple de 10 à 50 000 clones, plus généralement de 10 à 10 000 clones, encore plus préférentiellement de 50 à 5000 clones. Les clones sont généralement déposés, de façon ordonnée, sur un ou plusieurs supports, de façon à faciliter l'analyse des résultats d'hybridation. Le support peut être en verre, nylon, plastique, fibre, etc., de manière générale tout support solide adapté à l'étalement d'acides nucléiques. Le dépôt des banques sur les supports peut être réalisé par des techniques conventionnelles connues de l'homme du métier, qui sont décrites par exemples dans la demande internationale WO99/46403.

Les banques utilisées peuvent comprendre à la fois des clones d'acides nucléiques correspondant à des gènes dont le niveau d'expression est modifié dans des cellules en situation de dérégulation (marqueurs génétiques quantitatifs) et des clones d'acides nucléiques dont une partie au moins de la séquence correspond à la séquence de gènes épissés différentiellement dans des cellules en situation de dérégulation (marqueurs génétiques qualitatifs). Ainsi, les marqueurs génétiques peuvent être générés selon des approches différentes, puis mélangés pour obtenir une réponse aussi prédictive que possible.
A cet égard, il est également possible d'utiliser des banques comprenant des clones générés à partir de différentes situations de dérégulation. Ainsi, comme indiqué ci-avant, les situations de dérégulation peuvent être induites de différentes façons (augmentation de l'expression du gène p53, augmentation de l'expression du gène Rb, utilisation de populations cellulaires différentes, utilisation d'une cellule tumorale, etc.). Ces différentes situations de dérégulation n'induisent pas toujours exactement les mêmes événements génétiques, et donc pas toujours exactement les mêmes banques d'acides nucléiques selon l'invention. Même si chacune de ces banques peut être utilisée individuellement dans le cadre de la présente invention, il est également possible de les combiner entre elles sur un même support (ou sur des supports séparés), de manière à augmenter encore le nombre de marqueurs génétiques de la toxicité et ainsi améliorer la prédictibilité des méthodes de l'invention.

Un objet de la présente invention réside donc également dans une préparation d'acides nucléiques comprenant des marqueurs génétiques qualitatifs et quantitatifs caractéristiques de dérégulation(s) cellulaire(s). Un objet particulier réside dans une banque comprenant des marqueurs génétiques caractéristiques de différentes situations de dérégulation. L'invention a aussi pour objet tout support solide sur lequel au moins deux banques d'acides nucléiques caractéristiques de deux situations de dérégulation ont été déposées. A cet égard, l'invention concerne encore un procédé de préparation d'une puce à ADN utilisable pour le diagnostic du potentiel toxique d'un composé test, comprenant le dépôt, sur un support solide, d'une ou plusieurs préparations d'acides nucléiques caractéristiques de situation(s) de dérégulation.

Par ailleurs, selon un mode préféré de mise en oeuvre, on utilise des banques d'acides nucléiques affinées par sélection des clones au fur et à mesure de l'utilisation, en fonction de leur implication effective dans les phénomènes de toxicité. Les banques initiales peuvent en effet comprendre par exemple l'ensemble des clones caractéristiques des événements génétiques d'une situation de dérégulation. Puis, la mise en oeuvre du procédé de diagnostic de l'invention permet d'observer que certains clones n'hybrident jamais ou que très rarement avec les échantillons d'acides nucléiques testés. De tels clones peuvent éventuellement être éliminés de la banque, permettant de générer des banques plus spécifiques, et donc plus universelles et prédictives.

Un autre objet avantageux de l'invention réside donc dans une banque d'acides nucléiques comprenant des clones d'acides nucléiques correspondant à des événements génétiques communs à une situation de dérégulation et une situation de toxicité.

Un autre objet réside dans une méthode de préparation de banques de marqueurs génétiques de la toxicité, comprenant l'hybridation entre une population d'acides nucléiques dérivés de cellules en situation de dérégulation et une population d'acides nucléiques dérivés de cellules en situation de contrôle, l'isolement, à partir du produit de la réaction d'hybridation, de clones caractéristiques de la situation de dérégulation, et l'hybridation des clones obtenus avec un échantillon d'acides nucléiques dérivés de cellules en situation de toxicité. Avantageusement, le procédé ci-dessus comprend en outre une étape de sélection, comprenant l'hybridation de la banque avec différents échantillons d'acides nucléiques dérivés de cellules en situation de toxicité induite par des composés différents, et l'identification des clones de la banque hybridant le plus souvent avec lesdits échantillons.

De manière plus spécifique, la présente invention décrit à présent l'identification et la caractérisation de tels clones, utilisables comme marqueurs génétiques de la toxicité.

A cet égard, l'invention décrit notamment l'identification, la préparation, le clonage et la caractérisation de clones particuliers, dont les séquences sont représentées par les identifiants SEQ ID NOs :1 à 37. Ces clones sont utilisables comme marqueurs génétiques de la toxicité au sens de la présente invention. Ces clones ont été isolés par des méthodes qualitatives et comprennent des séquences correspondant à des altérations génétiques qualitatives caractéristiques d'événements de dérégulation affectant des gènes cellulaires variés, tels que des gènes codant pour des protéines, enzymes, gènes mitochondriaux, facteurs transcriptionnels, gènes pathologiques, gènes de réponse au stress, gènes impliqués dans la transduction de signaux, etc. De tels clones, les préparations et banques les contenant, ainsi que leurs utilisations, constituent des objets particuliers de l'invention.

Notamment, un objet particulier de l'invention réside dans une préparation ou une banque d'acides nucléiques comprenant une pluralité de clones d'acides nucléiques marqueurs génétiques de la toxicité, ladite préparation ou banque comprenant au moins un clone de séquence choisie parmi SEQ ID Nos : 1 à 37.

Un objet particulier de l'invention réside, plus généralement, dans toute banque d'acides nucléiques comprenant au moins un clone de séquence choisie parmi SEQ ID Nos : 1 à 37.

Un clone de séquence SEQ ID NO :X désigne au sens de l'invention (i) tout clone comprenant ladite séquence SEQ ID NO :X complète (ou une séquence complémentaire de celle-ci), le cas échéant bordée de séquences supplémentaires, de préférence un clone consistant en ladite séquence SEQ ID NO :X complète (ou une séquence complémentaire de celle-ci) ; ainsi que (ii) tout clone comprenant une partie seulement de la séquence SEQ ID NO :X (ou d'une séquence complémentaire de celle-ci), éventuellement bordée à une extrémité de séquence(s) supplémentaire(s) (notamment provenant de la séquence du gène ou messager correspondant), la partie de la séquence étant suffisante pour permettre une hybridation spécifique (par exemple au moins 10 bases) ; ou encore (iii) toute autre séquence nucléique, spécifique du gène ou d'un événement génétique de dérégulation du gène mis en évidence par la séquence SEQ ID NO :X, sans nécessairement chevaucher avec la séquence SEQ ID NO :X. Les séquences (iii) sont par exemple des séquences d'une autre région du gène concerné, permettant sa mise en évidence dans des conditions similaires. Les clones (i) à (iii) selon l'invention comportent typiquement entre 10 et 1000 bases, plus généralement entre 20 et 500 bases, dont une partie au moins est spécifique de l'événement génétique considéré.

Les préparations ou banques de l'invention comprennent plus préférentiellement au moins 5, de préférence au moins 10, plus préférentiellement au moins 15, encore plus préférentiellement au moins 20 clones de séquence choisie parmi SEQ ID Nos : 1 à 37. Comme indiqué ci-avant, il s'agit typiquement de banques comprenant entre 10 et 50 000 clones, plus généralement entre 10 et 5000 clones, notamment entre 50 et 1000 clones, dont une partie au moins est représentée par un ou plusieurs clones de séquence choisie parmi SEQ ID Nos : 1 à 37.

Les banques et préparations selon l'invention peuvent être déposées sur des supports et utilisées comme décrit dans la suite du texte.

L'une des applications majeures de l'identification et du clonage de ces marqueurs génétiques concerne l'évaluation du potentiel toxique d'un composé test. Cette évaluation peut s'effectuer en hybridant une sonde représentant les ARN messagers d'une culture cellulaire traitée par ce produit avec une ou plusieurs banques de signatures caractéristiques de situation(s) de dérégulation, telles que décrites ci avant. Une autre application réside dans l'identification d'altérations génomiques (notamment de SNPs) corrélées à une réponse d'un sujet à un composé donné, par exemple la réponse à un traitement, la sensibilité à un composé, etc. Ces applications sont décrites plus en détails dans ce qui suit.

### 5. Méthodes d'analyse et de diagnostic de la toxicité

L'invention a donc également pour objet une méthode pour l'analyse du potentiel toxique de composés tests, utilisant les marqueurs génétiques décrits ci avant. Notamment, l'invention permet de déterminer le potentiel toxique de tout composé par hybridation d'un échantillon d'acides nucléiques de cellules traitées par ce composé avec les marqueurs génétiques définis ci-dessus, le profil d'hybridation observé indiquant le potentiel toxique du composé. Dans ce but, les marqueurs génétiques utilisés sont préférentiellement regroupés sous forme de banques, afin de fournir une réponse aussi prédictive que possible. L'un des avantages de la présente invention réside également dans le nombre important de marqueurs utilisés, qui rend l'information générée d'autant plus prédictive. Le caractère prédictif de l'information est en outre consolidé par la nature des marqueurs utilisés et préparés.

Un objet particulier de l'invention réside dans une méthode d'analyse du potentiel toxique d'un composé test, comprenant au moins une étape d'hybridation entre a) un échantillon d'acides nucléiques de cellules traitées par ce composé et b) une banque d'acides nucléiques correspondant à des événements génétiques caractéristiques de situation(s) de dérégulation(s) de voie(s) de signalisation cellulaire, le profil d'hybridation indiquant le potentiel toxique du composé test. En particulier, plus le nombre de clones positifs à l'hybridation est élevé, plus le composé test est considéré comme potentiellement toxique.

L'analyse du profil toxique du composé est plus généralement faite en comparant le profil d'hybridation, sur la banque, d'un échantillon d'acides nucléiques de cellules traitées par ce composé avec celui d'un échantillon d'acides nucléiques de cellules non-traitées par ce composé. Cette comparaison permet de mettre en évidence une différence entre le nombre de clones hybridés dans la situation toxique et dans la situation contrôle, cette différence de nombre de clones permettant d'attribuer un index de toxicité au composé test.

Plus particulièrement, l'invention réside donc dans une méthode d'analyse du potentiel toxique d'un composé test, comprenant au moins
- une étape d'hybridation entre a) un échantillon d'acides nucléiques de cellules traitées par ce composé et b) une banque d'acides nucléiques correspondant à des événements génétiques caractéristiques de situation(s) de dérégulation(s) de voie(s) de signalisation cellulaire(s),
- une étape d'hybridation entre c) un échantillon d'acides nucléiques de cellules non traitées par ce composé et b) une banque d'acides nucléiques correspondant à des événements génétiques caractéristiques de situation(s) de dérégulation(s) de voie(s) de signalisation cellulaire,
- la comparaison des profils d'hybridation obtenus indiquant le potentiel toxique du composé test.

La mise en évidence du profil d'hybridation peut être réalisée par toute technique connue de l'homme du métier, et notamment par marquage des échantillons d'acides nucléiques tests et détection du marquage présent sur la banque. Ainsi, l'invention réside plus particulièrement dans une méthode d'analyse du potentiel toxique d'un composé test comprenant au moins l'hybridation séparée entre a) des sondes nucléiques marquées correspondant aux ARN de cellules non traitées et de cellules traitées par ledit composé test et b) une banque d'acides nucléiques correspondant à des événements génétiques (notamment transcriptionnels et/ou d'épissage) caractéristiques de dérégulation(s), le profil d'hybridation indiquant le potentiel toxique du composé test.

La marquage des acides nucléiques peut être réalisé par toute technique connue de l'homme du métier, tel que notamment l'emploi de marqueurs radioactifs, fluorescents, enzymatiques ou colorimétriques, par exemple. Généralement, le marquage est radioactif, et la mise en évidence d'une hybridation comprend la détection de la radioactivité présente sur le support. Le marquage peut bien entendu être fluorescent, et la mise en évidence d'une hybridation comprend dans ce cas la détection de la fluorescence émise sur le support.

Comme indiqué précédemment, dans les méthodes de l'invention, les banques d'acides nucléiques correspondant à des événements génétiques caractéristiques de dérégulation(s) peuvent être en particulier des banques d'acides nucléiques caractéristiques de situations de dérégulation de la croissance et/ou de la viabilité cellulaire. Plus particulièrement, il s'agit de banques d'acides nucléiques caractéristiques de cellules en situation de dérégulation de la croissance cellulaire, notamment de cellules transformées, en particulier de cellules tumorales.

Plus préférentiellement, dans les méthodes de l'invention telles que décrites ci-dessus, les banques d'acides nucléiques sont des banques d'acides nucléiques correspondant à des événements génétiques caractéristiques des altérations de voies de signalisation induites par l'activation, de préférence l'expression de tout ou partie d'un gène suppresseur de tumeur, de préférence de p53. Il s'agit en particulier de banques caractéristiques de l'apoptose induite par un gène suppresseur de tumeur, de préférence par p53. Les résultats présentés dans les exemples montrent en particulier que, grâce à l'emploi de la stratégie spécifique du criblage différentiel qualitatif, une situation d'apoptose consécutive à l'induction de l'expression de la protéine p53 entraîne de nombreuses modifications d'épissage, en plus de modifications d'ordre quantitatif liées à une régulation transcriptionnelle de différents gènes. L'invention montre que des sondes issues de cellules traitées par différents composés toxiques hybrident avec des clones des banques issues de criblages quantitatifs et qualitatifs réalisés à partir de cellules contrôles d'une part et de cellules dans lesquelles l'expression de la forme sauvage de p53 est induite d'autre part. Cela indique que des événements transcriptionnels et d'épissage sont partagés entre des situations toxiques et des situations où la protéine p53 est exprimée de façon forcée dans sa forme sauvage.
Par ailleurs, les résultats obtenus montrent également que, lorsque les cellules traitées sont du même type cellulaire que celles dans lesquelles a été induite l'expression de p53, les sondes réalisées à partir d'ARN messagers de cellules traitées par des doses croissantes de produits toxiques hybrident d'autant plus de clones au sein des banques d'évènements quantitatifs et qualitatifs que les doses sont élevées et donc la toxicité importante. Ceci montre qu'il existe une corrélation entre le niveau de toxicité et le profil d'hybridation sur les banques de l'invention.
Il est clair cependant que l'application des méthodes, kits et banques de l'invention n'est pas restreinte à un type particulier de cellules. Notamment elle n'est pas restreinte aux tests de produits réalisés sur les mêmes cellules que celles dont sont dérivés les clones et banques d'analyse quantitative ou qualitative. En effet, les résultats présentés montrent que les clones de l'invention permettent, de manière avantageuse, un diagnostic de la toxicité indépendant du composé toxique et du type cellulaire testé.

Dans un mode spécifique de mise en oeuvre, la banque d'acides nucléiques est une banque comprenant au moins un clone de séquence choisie parmi SEQ ID Nos : 1 à 37, telle que définie ci-avant.

Dans un mode particulier de mise en oeuvre des méthodes de l'invention les cellules traitées ou non traitées par le composé test et les cellules utilisées pour générer les banques sont de type différent.

Plus particulièrement, les cellules traitées ou non traitées par le composé test peuvent être d'origine et de type variés. Il s'agit préférentiellement de cellules mammifères, encore plus préférentiellement de cellules humaines. Il peut s'agir de cultures primaires ou de lignée cellulaires. Le type de cellules utilisé et leur concentration, densité et état physiologique (au repos, stimulé, etc.), peuvent être choisis par l'utilisateur des méthodes de l'invention, en fonction des composés tests et des applications envisagées. Par ailleurs, il peut également s'agir de cellules extraites d'organes ou de tissus d'animaux traités ou non traités par le composé.

Le composé test peut également être de nature très variée. Ainsi, il peut s'agir d'un composé isolé ou d'un mélange de substances. Le composé peut être de nature chimique ou biologique. Il peut s'agir notamment d'un peptide, polypeptide, acide nucléique, lipide, glucide, d'une molécule chimique, d'extraits végétaux, de banques combinatoires, etc. Le composé test peut également être un traitement appliqué aux cellules (radiation, UV, etc.). Pour la mise en oeuvre du procédé de l'invention, le composé test peut être appliqué à différentes concentrations, choisies par l'utilisateur. Par ailleurs, l'échantillon d'acides nucléiques dérivé des cellules traitées peut être préparé à des instants variables après traitement par le composé. Ainsi, des cinétiques peuvent être réalisées, le cas échéant.

Les acides nucléiques et les sondes correspondantes sont préparés par les techniques conventionnelles. L'hybridation peut ensuite être réalisée, également dans des conditions qui peuvent être adaptées par l'homme du métier et l'utilisateur. A cet égard, l'hybridation peut être réalisée en condition de stringence élevée, moyenne ou faible, selon le degré de sensibilité recherché, la quantité de matériel disponible, etc. En outre, selon la présente invention, les hybridations entre la banque et les sondes peuvent être homologues (lorsque la banque et les sondes sont constituées à partir d'acides nucléiques de la même espèce, par exemple humaine) ou hétérologues (lorsque la banque et les sondes sont constituées à partir d'acides nucléiques d'une espèce différente, par exemple une banque d'origine humaine et des sondes préparées à partir de matériel d'un autre mammifère). Bien que les banques et sondes soient préférentiellement construites à partir de matériel humain, il est en effet possible d'utiliser d'autres sources, notamment pour les sondes (par exemple un matériel murin). Dans le cas de telles hybridations hétérologues, les conditions d'hybridation peuvent être adaptées par l'homme du métier, selon les techniques conventionnelles, notamment en diminuant la température d'hybridation et/ou en augmentant la salinité de la solution d'hybridation.

### 6. Détermination d'un index de toxicité

Un avantage des méthodes de l'invention réside dans la possibilité de déterminer un index de toxicité d'un produit donné sur tout type cellulaire. Pour cette application, les clones qui correspondent à des éléments identifiés à partir des cellules qui ont servi à établir les banques spécifiques de la viabilité cellulaire aussi bien que ceux spécifiques de la dérégulation induite sont hybridés avec une sonde dérivée des cellules non traitées à étudier. Leur hybridation constitue le motif de référence d'expression d'ARN commun entre le modèle de dérégulation et le modèle cellulaire étudié. Une comparaison de l'expression des ARN de la situation cellulaire non traitée avec ceux de situations traitées par différents produits à différentes concentrations et à différents temps de traitement est effectuée aisément par comparaison des profils d'hybridation de chaque sonde issue de chaque situation étudiée. L'index de toxicité d'une situation peut être évalué en assignant une valeur 1 aux clones hybridés avec la sonde non traitée et non-hybridés avec la situation traitée d'intérêt d'une part, et en assignant également la valeur 1 aux clones non hybridés avec la sonde non traitée mais hybridés avec la sonde traitée d'autre part. L'index est alors la somme des valeurs 1 individuelles exprimé en pourcentage du nombre de clones soumis à l'hybridation. Cet index est donc susceptible de varier entre 0 et 100. Cet exemple de calcul d'index n'est pas limitatif. D'autres méthodes mathématiques, algorithmes ou processus de calculs, tels des réseaux neuronaux pourront également être utilisés.

L'intérêt de déterminer un tel index en hybridant des clones dérivés d'un criblage différentiel qualitatif entre deux états d'un même type cellulaire (en prolifération et induit en apoptose) avec des sondes issues d'autres types cellulaires est avéré par la possibilité de déterminer un index d'autant plus important pour un composé donné que la dose appliquée aux cellules testées est forte.
La valeur de l'index est naturellement dépendante du seuil à partir duquel un clone est considéré comme devant recevoir la valeur 1 ou 0. L'index est d'autant plus important que le facteur de répression ou d'induction que doit présenter un clone pour être considéré est faible. La robustesse de la méthode est néanmoins attestée par le fait qu'il existe une linéarité entre le facteur retenu et l'index de toxicité calculé. Cette linéarité est le reflet du nombre d'événements indépendants qui constituent la banque qualitative qui permet de s'affranchir du poids trop important d'un clone particulier ayant un différentiel d'expression entre situation traitée ou non traitée trop important.

### 7. Recherche de polymorphismes associés à une toxicité pour un composé donné :

La détermination du potentiel toxique d'un composé selon la procédure décrite en 5 et dans les exemples permet d'identifier des séquences qui dérivent de gènes impliqués dans des mécanismes de stress et de mort cellulaire dont l'expression est affectée par ce composé.
Ces gènes représentent des candidats de choix pour rechercher des polymorphismes susceptibles d'affecter la réponse au composé étudié. Ces polymorphismes permettent de ségréguer les patients candidats pour l'administration de ce composé en groupes plus ou moins susceptibles de développer une toxicité exacerbée, hépatique notamment.
Par exemple, il peut facilement être envisagé qu'un polymorphisme qui affecte un gène dont l'expression est réprimée par un composé rende un patient plus susceptible à l'action toxique de ce composé.
Sans considérer ce cas particulier, l'importance des polymorphismes de cette sélection de gènes réalisée au moyen de l'invention est évaluée en établissant les corrélations qui existent entre ces polymorphismes et les toxicités observées lors des essais cliniques effectués sur le composé étudié.

L'invention a donc pour objet l'utilisation des clones ou acides nucléiques décrits, pour l'identification de mutations ou polymorphismes, notamment de SNPs, correlés à la réponse de sujets à des composés pharmaceutiques.

L'invention concerne, plus généralement, l'utilisation d'acides nucléiques représentatifs d'événements d'épissages caractéristiques d'une situation physiopathologique donnée, pour l'identification ou la caractérisation de SNPs correlés à ladite situation.

L'identification peut être réalisée selon différentes méthodes ou approches connues de l'homme du métier. Ainsi, elle peut être réalisée par séquençage, à partir d'échantillons biologiques provenant de sujets répondeurs et non-répondeurs à un composé, des gènes correspondant aux clones ou acides nucléiques sélectionnés, et recherche de polymorphismes dans ledist gènes. L'identification peut également être réalisée à partir de banques de SNPs, par recherche de SNPs présents dans lesdits gènes.

L'invention concerne également une méthode d'identification de SNPs ou autres mutations ou polymorphismes permettant d'évaluer la réponse d'un sujet à un composé donné, comprenant (i) l'identification in vitro d'acides nucléiques caractéristiques d'événements d'épissages induits dans une cellule traitée par ledit composé et (ii) l'identification de SNPs ou autres mutations ou polymorphismes dans le ou les gènes correspondant à des acides nucléiques identifiés en (i), lesdits SNPs ou autres mutations ou polymorphismes permettant d'évaluer la réponse d'un sujet au composé donné. L'invention fournit ainsi des méthodes et moyens pour sélectionner des gènes pour lesquels des polymorphismes sont associés à une toxicité pour un composé donné.

L'invention concerne également une population d'acides nucléiques spécifiques de polymorphismes ou SNPs ainsi identifiés, notamment d'amorces nucléotidiques pour l'amplification sélective des polymorphismes, ou de sondes pour l'hybridation sélective avec les polymorphismes considérés.

L'invention concerne également des méthodes pour l'analyse (par exemple la prédiction, l'évaluation ou la détermination) de la réponse d'un sujet à un composé test, comprenant l'étude de polymorphismes, notamment l'analyse de SNPs, tels que définis ci-avant. Une méthode particulière concerne l'étude de la sensibilité ou de la réponse d'un sujet à un composé, notamment à la toxicité d'un composé, comprenant l'analyse, à partir d'un échantillon biologique dudit sujet comprenant de l'ADN, de la présence dans l'ADN du sujet, de polymorphismes, SNPs ou autres altérations génomiques présents dans des gènes dont l'épissage est modifié en réponse audit composé. Comme indiqué ci-avant, la présence de polymorphismes, SNPs ou autres altérations génomiques dans l'ADN d'un sujet peut êre déterminée par différentes techniques connues de l'homme du métier, comme le séquençage, l'amplification, l'hybridation, des méthodes séparatives, chromatographiques, etc. Une approche préférée consiste à amplifier l'ADN du sujet au moyen d'un amorce spécifique de l'altération (notamment du SNP) recherchée. Une autre approche consiste à utiliser une sonde nucléotidique dont la séqunce est spécifique de l'altération (notamment du SNP) recherchée. Il est entendu que l'invention n'est pas limitée à des techniques particulières.

### 8. Kits

La présente demande a également pour objet des kits (ou trousses) pour la mise en oeuvre d'une méthode d'analyse telle que décrite ci avant. Plus particulièrement, l'invention a pour objet des kits pour l'étude du potentiel toxique d'un composé test, comprenant au moins :
- une banque d'acides nucléiques correspondant à des événements génétiques (transcriptionnels et/ou d'épissage) caractéristiques d'altération(s) d'une ou plusieurs voies de signalisation cellulaire, notamment de situation(s) de dérégulation de la croissance et/ou de la viabilité cellulaire.

Plus généralement, les kits de l'invention comprennent une banque de marqueurs génétiques caractéristiques de dérégulation(s), par exemple de l'apoptose, telle que définie dans l'invention, déposés sur un support solide.

Plus préférentiellement, les kits de l'invention comprennent une banque d'acides nucléiques caractéristiques de plusieurs situations de dérégulation induites dans des conditions différentes.

Selon un autre mode particulier, les kits de l'invention comprennent au moins une banque d'acides nucléiques comprenant des clones correspondant à des événements génétiques communs à des cellules en situation de dérégulation et en situation de toxicité.

Selon un mode particulier, l'invention concerne tout kit comprenant une banque d'acides nucléiques, la banque comprenant au moins un clone de séquence choisie parmi SEQ ID Nos : 1 à 37, telle que définie ci-avant. La banque est avantageusement déposée sur un support, de préférence solide, notamment en verre, silice, filtre, membrane, nylon, plastique, etc.

Dans les kits de l'invention, les banques comprennent en outre avantageusement des clones témoins, permettant d'étalonner les signaux détectés. Ces témoins peuvent être plus particulièrement constitués de trois types de matériel :
- des clones d'ADN génomique,
- les ADNc servant à la confection des sondes qui hybrideront les banques, et/ou
- des ADNc correspondant à des ARNm codant pour des protéines contrôles, comme par exemple des protéines (notamment des enzymes) de ménage telles que la β-actine et la GAPDH (Glycéraldéhyde Phosphate DésHydrogénase).
Les contrôles fournis par les enzymes de ménage sont utilisés classiquement par l'homme du métier lors d'expérience de « cDNA display ». Cependant, lors de phénomènes de dérégulation selon l'invention, la quantité d'ARNm de ces enzymes peut varier. Aussi, dans le but de normaliser le pouvoir hybridant (et donc le signal) de chaque sonde, la présente invention propose d'utiliser un ou plusieurs autres systèmes de témoin. A cet égard, l'utilisation d'ADN génomique et/ou des ADNc (non marqués) correspondant à la sonde (c'est-à-dire à la population d'ADN de l'échantillon biologique traité ou non traité) permet, selon l'invention, d'obtenir des témoins d'hybridation indépendants de toute variation qualitative ou quantitative de l'expression génique. Des mêmes quantités d'ADNc douées de la même activité spécifique après marquages (radioactifs) doivent en principe générer des signaux équivalents en hybridant d'une part avec l'ADN génomique et d'autre part avec elles-mêmes.

Les kits de l'invention peuvent comprendre en outre :
- des oligonucléotides pour la préparation des sondes, et/ou
- un protocole pour la réaction d'hybridation, et/ou
- des éléments et informations permettant l'établissement d'un index de toxicité pour chaque produit testé, notamment un logiciel de traitement du signal.

Les oligonucléotides sont préférentiellement du type semi-aléatoire, comme par exemple les oligonucléotides de formule X-Nₙ-AGGT, où X est une séquence stabilisatrice telle que GAGAAGCGTTATG ou CCACGCTACAAG(C), N est une base, et n est un nombre entier compris entre 3 et 8 inclus.

La faisabilité, la réalisation et d'autres avantages de l'invention sont illustrés plus en détails dans les exemples qui suivent, qui doivent être considérés comme illustratifs et non limitatifs.

### Légende des figures :

Figure 1. Hybridation différentielle de sondes provenant de cellules HepG2 non traitées (A), traitées à l'ethanol (B) ou traitées à la cyclosporine sur des filtres comportant des fragments PCR correspondant aux clones issus du criblage qualitatif d'un système d'apoptose induite par p53.

Figure 2. Index de toxicité calculé à partir de différents composés toxiques sur le filtre apoptose p53.

Figure 3. Hybridation différentielle de sondes provenant de cellules HepG2 non traitées (A), traitées au clenbuterol (B), traitées au R propranolol (C) ou traitées au DL propranolol (D) sur des filtres comportant des fragments PCR correspondant aux clones issus du criblage qualitatif à partir de biopsies tumorales.

### 9. Exemples

### 9.1. Identification de marqueurs génétiques de toxicité à partir d'un criblage qualitatif sur un système cellulaire d'apoptose.

Un exemple de l'apport du criblage différentiel qualitatif à l'identification de marqueurs génétiques associés à la toxicité de molécules est illustré par l'application de DATAS à un modèle d'induction d'apoptose par induction de l'expression de la forme sauvage de p53. Ce modèle cellulaire a été établi par transfection d'un système d'expression inductible pour le gène suppresseur de tumeur p53. De façon à identifier les différences qualitatives qui sont associées spécifiquement à l'apoptose induite par p53, un criblage qualitatif a été mis en oeuvre à partir des ARN messagers extraits de cellules induites (ml) et non induites (mNI). Ces ARN messagers sont convertis en ADN complémentaires (cl) et (cNI) à l'aide de reverse transcriptase (RT) et d'oligonucléotides amorces biotynilés. Des hybrides ml/cNI et cl/mNI sont ensuite réalisés en phase liquide.

Les quantités requises d'ARN et d'ADNc (en l'occurrence 200ng de chaque) sont combinées et précipitées à l'ethanol. Les échantillons sont repris dans un volume de 30µl dans un tampon d'hybridation (Hepes (pH 7.2) 40 mM, NaCl 400mM, EDTA 1mM) supplémenté de formamide désionisée (80% (v/v). Après dénaturation 5 min à 70°C, les échantillons sont incubés sur la nuit à 40°C.

Les billes Streptavidine (Dynal) sont lavées puis reconditionnées dans un tampon de fixation (2X= Tris-HCl (pH 7,5) 10 mM, NaCl 2M, EDTA 1 mM). Les échantillons d'hybridation sont amenés à un volume de 200 µl avec de l'eau puis ajustés à 200 µl de billes pour une incubation de 60 min à 30°C. Après capture sur aimant et lavages des billes, celles-ci sont reprises dans 150 µl de tampon RnaseH puis incubées pendant 20 min à 37°C. Après capture sur aimant, les régions non hybridées ont été relarguées dans le surnageant qui est traité à la Dnase puis extrait au phénol acide / chloroforme puis précipité à l'éthanol. Les précipitations à l'éthanol de faibles quantités d'acides nucléiques sont effectuées à l'aide d'un polymère commercial SeeDNA (Amersham Pharmacia Biotech) permettant de récupérer de façon quantitative des acides nucléiques à partir de solutions très diluées (de l'ordre du ng/ml).

La synthèse d'ADNc à partir des échantillons d'ARNs provenant de l'action de la RnaseH est effectuée à partir d'hexanucléotides aléatoires à l'aide de Superscript Il Reverse Transcriptase. L'ARN est ensuite dégradé à l'aide d'un mélange de RnaseH et de Rnase T1. L'amorce, les nucléotides non incorporés et les enzymes sont séparés de l'ADNc à l'aide d'une cartouche « GlassMAX Spin ». L'ADNc correspondant aux boucles d'épissage est ensuite soumis à une réaction de PCR en utilisant des oligonucléotides de type semi-aléatoire de structure : (FS3)N7AGGT, où (FS3) = CCACGCTACAAG.

La réaction de PCR est réalisée à l'aide de Taq Polymérase sur 30 cycles :
- Dénaturation initiale : 94°C pdt 1 min.
- 94°C pdt 30 s
- 55°C pdt 30s
- 72°C pdt 30s
- Extension finale : 72°C pdt 5 min.

Les produits de PCR sont clonés dans le vecteur pGEM-T (Proméga) possédant un T flottant aux extrémités 3' afin de faciliter le clonage de fragments issus de l'activité de la Taq Polymérase. Après transformation dans les bactéries JM109 compétentes (Proméga), les colonies obtenues sont stockées dans des plaques 96 puits. Des produits de PCR générés à l'aide d'oligonucléotides T7 et Sp6 situés sur le plasmide de clonage et encadrant les inserts sont déposés sur des filtres de nitrocellulose.

Des cellules HepG2 sont ensuite traitées individuellement par différents composés de potentiel toxique variable. Dans cet exemple, la dose utilisée est calculée par un test de viabilité cellulaire (MTT) et correspond à une IC80. Les doses des différents composés ainsi que les durées des traitements sont résumées sur le tableau 1 suivant:

**Tableau 1 :**

| Composé | Concentration | Temps |
|---|---|---|
| Aspirine | 2mM | 18h |
| Paracetamol | 1 mM | 18h |
| Cyclosporine | 2,5µM | 18h |
| Diclofenac | 50µg/ml | 18h |
| Beclomethazone | 50nM | 18h |
| Methotrexate | 500nM | 18h |
| Erythromicyne | 2,5µg/ml | 18h |
| Vinblastine Sulfate | 30µM | 18h |
| Clonidine | 150µM | 18h |
| Valinomycine | 15 nM | 18h |
| Etoposide | 50µg/ml | 18h |
| Camptothecine | 1µg/ml | 4h |
| PMA | 1µg/ml | 18h |
| Ethanol | 0,1M / 0,5M | 4h |
| 5 FU | 10µM | 24h |
| Cycloheximide | 5µg/ml | 18h |
| FCCP | 5µM | 18h |
| Staurosporine | 50nM | 18h |
| Terbutaline Sulfate | 200ng/ml | 18h |
| Epinephrine Hydrate | 50µg/ml | 18h |
| AZT | 50µg/ml | 18h |
| H2O2 | 0,1 mM | 18h |
| Actinomycin D | 0,02µg/ml | 18h |
| | | |

Des sondes marquées correspondant aux ARN sont réalisées à partir de ces situations. Pour cela, le cDNA premier brin obtenu à partir d'ARN total selon les techniques connues de l'homme de métier sert de matrice pour une réaction de PCR à l'aide des oligonucléotides (FS4)GN3AGGT et FS3CN3AGGT, dans lesquels (FS4) = GAGAAGCGTTAT, selon le programme suivant :

| | |
|---|---|
| Dénaturation initiale | 95°C, 5 min |
| 5 cycles avec | dénaturation : 94°C, 30 sec |
| | appariement : 30°C, 30 sec |
| | montée en température de 30 à 72°C en 30 sec |
| | élongation : 72°C, 30 sec |
| 35 cycles avec | dénaturation : 94°C, 30 sec |
| | appariement : 57°C, 30 sec |
| | élongation : 72°C, 30 sec |
| Elongation finale | 72°C, 7 min |

Les produits PCR sont marqués à l'aide du kit Redyprime (Amersham).

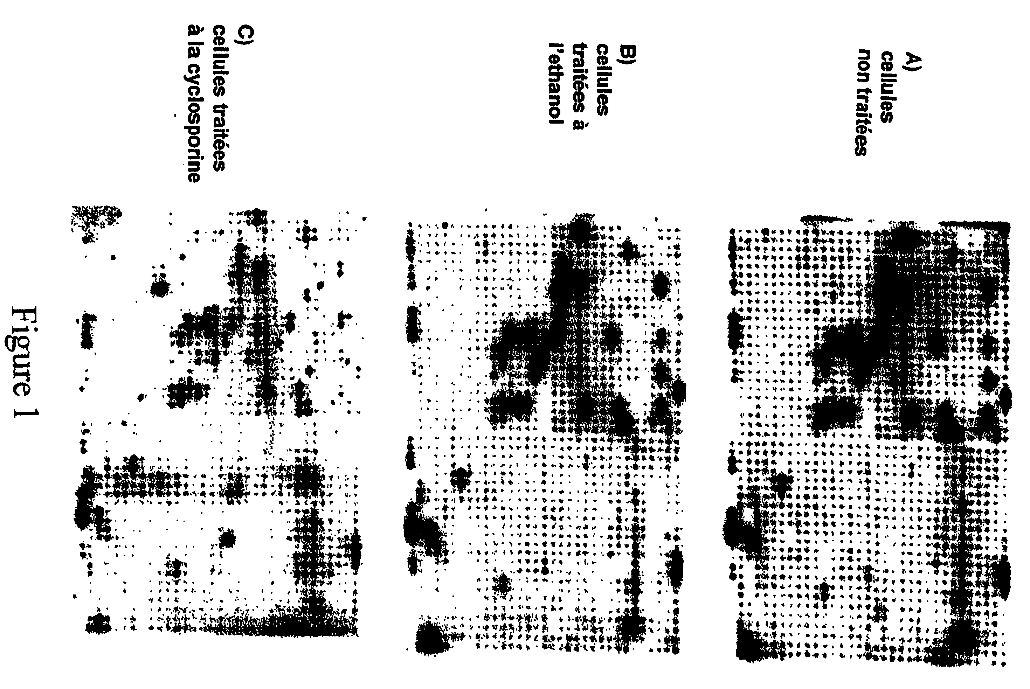

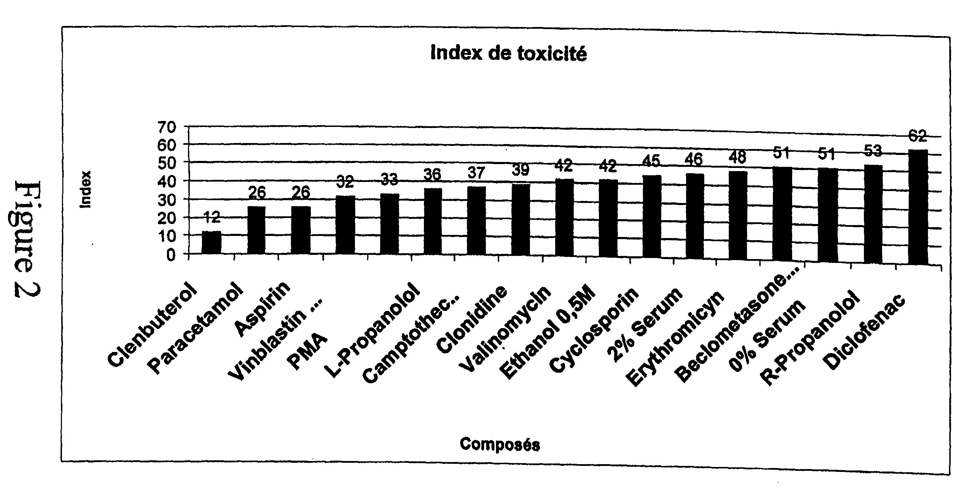

Les filtres sont mis à pré-hybrider dans un tampon d'hybridation (Rapid Hybrid Buffer, Amersham) contenant du sperme de saumon (100 µg/ml) pendant 30 min à 65°C. La sonde PCR est ensuite ajoutée sur la membrane (0.5x10⁶ à 1x10⁶ cpm/ml) pour incubation de 2 à 18 heures à 65°C. Les filtres sont lavés en tampon 0,5X SSC, 0,1% SDS pendant 30 min à 65°C puis en tampon 0,2X SSC, 0,1% SDS. Les profils d'hybridations sont analysés par mesure de la radioactivité sur Instantlmager (Packard Instruments) (Figure 1). La quantification des intensités individuelles d'hybridation permet le calcul d'un index selon le procédé décrit précédemment (Figure 2). Ce résultat indique qu'il est possible de classifier différents produits selon leur capacité à induire l'expression de marqueurs épissés de façon différentielle lors d'une toxicité induite par p53. Ainsi le diclofénac mobilise davantage d'événements d'épissage identiques à ceux induits lors de la toxicité liée à l'expression de p53 que l'aspirine, dans les cellules HepG2. L'utilisation de banques qui regroupent des séquences d'ADN complémentaires épissées de façon différentielle entre une situation (saine) de référence et une situation mobilisant une voie majeure de toxicité permet donc de classifier des produits dont les actions ne sont pas distinguables par les tests classiques de toxicité tels le test MTT.

### 9.2. Identification de marqueurs génétiques de toxicité à partir d'un criblage qualitatif sur des biopsies tumorales

Un deuxième exemple d'utilisation de l'invention dans l'identification de marqueurs génétiques de toxicité consiste en la génération de banques d'acides nucléiques différentiels entre tissus sains et tissus tumoraux. L'intérêt d'identifier les séquences spécifiquement exprimées dans les tissus contrôle (sains) d'une part et dans les tissus tumoraux d'autre part tient au fait qu'au sein d'une tumeur sont présentes, dans différentes cellules mais également au sein de mêmes cellules, des informations caractéristiques de croissance dérégulée et de mort cellulaire. Par rapport à un modèle d'apoptose induite par expression dérégulée de p53 ou toute autre protéine ayant un impact négatif sur la croissance et la viabilité cellulaire, l'utilisation de tumeurs permet donc de disposer en plus, sur des filtres à visée de toxicologie prédictive, des séquences dont l'expression est caractéristique d'hyper prolifération ou de carcinogénicité.

Des biopsies tumorales provenant de 7 patients ayant développé un cancer tête et cou, ainsi que des biopsies de tissus sains provenant de la luette des même patients, ont servi de matériel de travail. La même procédure que celle décrite dans l'exemple 9.1 a été utilisée si ce n'est une adaptation quantitative prenant en compte les faibles quantités de matériel disponibles dans des biopsies d'origine humaine.

Les fragments d'acides nucléiques générés par l'application de l'invention aux tissus tumoraux et normaux ont été disposés sur filtres de nitrocellulose. Ces filtres ont été hybridés avec les même sondes radioactives que celles utilisées dans l'exemple 8.1, c'est à dire générées à partir du matériel génétique de cellules HepG2 traitées par différents composés de potentiel toxique variable. Des profils d'hybridation différentiels ont été observés entre les différents composés, démontrant ainsi l'intérêt d'une utilisation de ces banques d'acides nucléiques pour l'évaluation d'une toxicité associée à un composé donné.

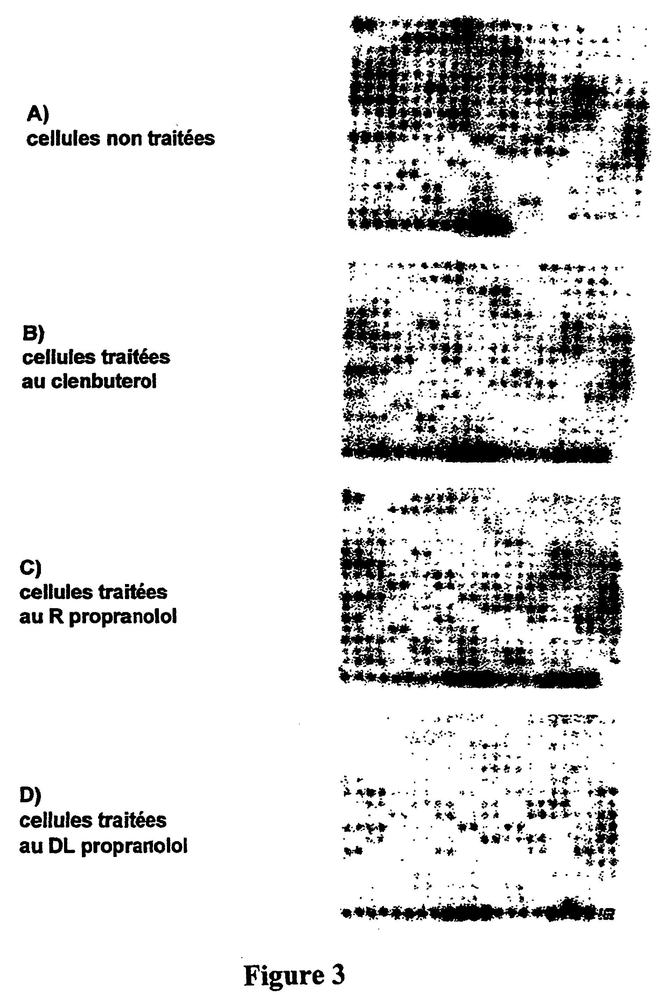

La figure 3 montre les signaux d'hybridations obtenus avec les sondes dérivées des cellules HepG2 normales, traitées au clenbutérol, à l'énantiomère inactif du propranolol ou encore avec un mélange racémique d'énantiomères actif et inactif du propranolol. Les doses de composés sont indiquées dans le tableau 1 précédent et la réalisation des sondes et les protocoles suivis pour les hybridations sont identiques à ce qui est mentionné dans le paragraphe décrivant l'utilisation des filtres rassemblant des signatures spécifiques du modèle d'apoptose induite par p53 (exemple 9.1.).

Les signaux d'hybridation présentés sur la figure 3 peuvent être quantifiés et servir de base à la détermination d'un index de toxicité pour chacun des composés selon le même mode opératoire que celui qui a permis d'obtenir les index présentés en figure 2. Ainsi, les hybridations présentées en figure 3 montrent que les effets de différents composés sur cellules HepG2, qui ne sont pas discernables par des techniques classiques employées en études toxicologiques comme la coloration au MTT, peuvent être classifiés selon leur capacité à mobiliser des événements d'épissage spécifiques des différences qui existent entre des cellules de tissu sain et des cellules tumorales.

### 9.3. Description de marqueurs génétiques de toxicité

La mise en oeuvre des techniques décrites aux exemples 9.1 et 9.2 a permis la constitution et l'utilisation de banques de clones, marqueurs génétiques de la toxicité. Les clones composant ces banques ont été analysés et séquencés, et la séquence d'une partie d'entre-eux est présentée dans la liste de séquences incluse à la présente demande (SEQ ID Nos : 1-37).

L'analyse de ces clones montre qu'ils correspondent à des événements génétiques qualitatifs affectant des gènes cellulaires très variés, ce qui confirme (i) l'efficacité des méthodes de l'invention, (ii) l'existence de marqueurs génétiques « universels » au sens de l'invention et (iii) l'importance de la diversité des clones pour obtenir une réponse prédictive.

Ainsi, les séquences identifiées permettent de mettre en évidence, par hybridation, des variations d'expression ou des altérations des gènes cellulaires humains suivants: Aldolase A (exon 9) (SEQ ID NO :1) ; Sous-Unité S4 du protéasome 26S (SEQ ID NO :2) ; Alpha-tubuline (SEQ ID NO :3) ; Glucosidase II (SEQ ID NO :4) ; Homologue du récepteur de la lamin B (SEQ ID NO :5) ; EF1-alpha (SEQ ID NO :6) ; Fra-1 (SEQ ID NO :7) ; Récepteur de tyrosine kinase AX1 (SEQ ID NO:8) ; Protéine spliceosomale SAP62 (SEQ ID NO :9) ; TRAF-3 (SEQ ID NO :10) ; EF2 (SEQ ID NO :11) ; TEF-5 (SEQ ID NO :12) ; CDC25b (SEQ ID NO :13) ; Kinase associée au récepteur de l'interleukine-1 (« IRAK ») (SEQ ID NO :14) ; WAF-1 (SEQ ID NO :15) ; c-fos (exon 4) (SEQ ID NO :16) ; ckshs1 (SEQ ID NO :17) ; PL16 (SEQ ID NO :18) ; NFAR-2 (SEQ ID NO:19) ; phosphatidylinositol4-kinase (SEQ ID NO :20), ERF (SEQ ID NO :21), tyrosine kinase du récepteur de type Eph (hEphB1b) (SEQ ID NO :22) ; protéine BAF60b du complexe SWI/SNF (SEQ ID NO :23) ; EB1 (SEQ ID NO :24) ; MSS1 (SEQ ID NO :25) ; récepteur alpha de l'acide rétinoïque (RARa) (SEQ ID NO :26) ; facteur d'initiation de la traduction eiF4A (SEQ ID NO :27) ; kinase de type STE20 (SEQ ID NO :28) ; protéine HSP 90kda (SEQ ID NO :29) ; Lipocortine Il (SEQ ID NO :30) ; protéine TPT1 (« translationally controlled tumor proteon ») (SEQ ID NO:31); Hsc70 (SEQ ID NO :32); Cytokeratine 18 (SEQ ID NO:33) ; 2-oxoglutarate dehydrogenase (SEQ ID NO :34) ; gène mitochondrial NADH6 (SEQ ID NO :35) ; gène mitochondrial NADH deshydrogenase 4 (SEQ ID NO :36) ; Sous-unité alpha de l'ATP synthase mitochondriale (SEQ ID NO :37).

Un objet particulier de l'invention réside notamment dans l'utilisation d'une sonde nucléique spécifique de tout ou partie d'un gène codant pour une protéine ou un facteur tel que mentionnés ci-dessus, pour la détection ou l'évaluation du potentiel de toxicité d'un composé, et pour la détection d'une situation de toxicité dans un échantillon biologique. Préférentiellement, la sonde comprend moins de 1000 bases, plus particulièrement moins de 500 bases, et comprend une séquence complémentaire d'une partie d'un gène mentionné ci-avant. Plus préférentiellement, la sonde est marquée.

Avantageusement, l'invention concerne un ensemble de sondes, notamment de 5 sondes ou plus, préférentiellement de 10 sondes ou plus, chacune étant complémentaire d'une partie d'un gène choisi parmi les gènes mentionnés ci-avant. Encore plus préférentiellement, les sondes sont immobilisées sur un support, comme décrit dans la présente demande.

## Revendications

1. Méthode d'analyse du potentiel toxique d'un composé test, comprenant au moins une étape d'hybridation entre a) un échantillon d'acides nucléiques de cellules traitées par ce composé et b) une banque d'acides nucléiques, ladite banque comprenant des clones spécifiques de (portions de) gènes ou transcrits caractéristiques de situation d'apoptose, le profil d'hybridation indiquant le potentiel toxique du composé test.

2. Méthode d'analyse du potentiel toxique d'un composé test selon la revendication 1, comprenant au moins l'hybridation séparée entre a) des sondes nucléiques marquées correspondant aux ARN de cellules non traitées et de cellules traitées par ledit composé test et b) une banque d'acides nucléiques, ladite banque comprenant des clones spécifiques de (portions de) gènes ou transcrits caractéristiques de situation d'apoptose, le profil d'hybridation indiquant le potentiel toxique du composé test.

3. Méthode selon la revendication 1 ou 2 **caractérisée en ce que** les sondes nucléiques a) correspondent aux ARN messagers des cellules traitées et non traitées.

4. Méthode selon l'une des revendications 1 à 3 **caractérisée en ce que** les sondes nucléiques a) sont des ADNc ou des fragments d'ADNc préparés à partir des ARNs des cellules traitées et non traitées.

5. Méthode selon l'une quelconque des revendications précédentes, **caractérisée en ce que** les sondes nucléiques a) sont des produits d'amplification.

6. Méthode selon l'une quelconque des revendications précédentes, **caractérisée en ce que** les sondes nucléiques a) sont marquées par des marqueurs radioactifs, fluorescents, enzymatiques ou colorimétriques.

7. Méthode selon l'une quelconque des revendications précédentes, **caractérisée en ce que** le composé test est un composé de nature chimique ou biologique.

8. Méthode selon l'une quelconque des revendications précédentes, **caractérisée en ce que** la banque b) comprend des acides nucléiques correspondant à des gènes dont le niveau d'expression est modifié dans une situation d'apoptose.

9. Méthode selon l'une des revendications 1 à 7, **caractérisée en ce que** la banque b) comprend des acides nucléiques dont une partie au moins de la séquence correspond à la séquence de gènes épissés différentiellement lors de situation d'apoptose.

10. Méthode selon la revendication 1 ou 2 **caractérisée en ce que** la banque b) comprend des acides nucléiques selon la revendication 8 et des acides nucléiques selon la revendication 9.

11. Méthode selon la revendication 9, **caractérisée en ce que** la banque b) est préparée par hybridation entre une population d'acides nucléiques d'une cellule en situation d'apoptose cellulaire et une population d'acides nucléiques d'une cellule en situation de contrôle, et isolement, à partir des hybrides formés, des acides nucléiques correspondant à des épissages différentiels.

12. Méthode selon l'une quelconque des revendications précédentes, **caractérisée en ce que** la situation de dérégulation est induite par modification de l'activation, préférentiellement de l'expression d'un gène initiateur ou exécuteur de l'apoptose.

13. Méthode selon la revendication 12, **caractérisée en ce que** la situation de dérégulation est provoquée par induction ou augmentation de l'activation, préférentiellement de l'expression d'un gène anti-oncogène.

14. Méthode selon la revendication 13, **caractérisée en ce que** le gène anti-oncogène est choisi parmi p53, Rb, p73, myc, TUPRO-2 et NHTS.

15. Méthode selon la revendication 12, **caractérisée en ce que** la situation de dérégulation est induite par modification de l'activation, préférentiellement de l'expression d'un gène impliqué dans la croissance ou la viabilité cellulaire.

16. Méthode selon la revendication 12, **caractérisée en ce que** la situation de dérégulation est induite par activation, préférentiellement expression constitutive ou inductible de tout ou partie d'un gène impliqué dans la croissance cellulaire, la viabilité cellulaire ou l'apoptose.

17. Méthode selon l'une des revendications 1 à 7, **caractérisée en ce que** la banque b) comprend au moins un clone de séquence choisie parmi SEQ ID Nos : 1 à 37, plus préférentiellement au moins 5.

18. Méthode selon l'une des revendications 1 à 7, **caractérisée en ce que** la banque b) comprend un ensemble de sondes, notamment de 5 sondes ou plus, préférentiellement de 10 sondes ou plus, chacune des sondes étant complémentaire d'une partie d'un gène choisi parmi les gènes Aldolase A; Sous-Unité S4 du protéasome 26S ; Alpha-tubuline ; Glucosidase II ; Homologue du récepteur de la lamin B; EF1-alpha ; Fra-1 ; Récepteur de tyrosine kinase AX1 ; Protéine spliceosomale SAP62 ; TRAF-3 ; EF2 ; TEF-5 ; CDC25b; Kinase associée au récepteur de l'interleukine-1 (« IRAK »); WAF-1 ; c-fos (exon 4); ckshs1 ; PL16 ; NFAR-2 ; phosphatidylinositol4-kinase, ERF, tyrosine kinase du récepteur de type Eph (hEphB1b) ; protéine BAF60b du complexe SWI/SNF ; EB1 ; MSS1 ; récepteur alpha de l'acide rétinoïque (RARa) ; facteur d'initiation de la traduction eiF4A ; kinase de type STE20 ; protéine HSP 90kda ; Lipocortine II ; protéine TPT1 (« translationally controlled tumor proteon »); Hsc70 ; Cytokeratine 18 ; 2-oxoglutarate dehydrogenase ; gène mitochondrial NADH6 ; gène mitochondrial NADH deshydrogenase 4 ; Sous-unité alpha de l'ATP synthase mitochondriale.

19. Méthode selon l'une quelconque des revendications précédentes, **caractérisée en ce que** les cellules traitées ou non traitées a) et en situation de dérégulation b) sont de type différent.

20. Méthode selon l'une quelconque des revendications précédentes, **caractérisée en ce que** les cellules traitées ou non traitées sont des cellules mammifère, de préférence humaines.

21. Méthode selon l'une quelconque des revendications précédentes, **caractérisée en ce que** les cellules traitées ou non traitées sont des lignées cellulaires.

22. Méthode selon l'une quelconque des revendications précédentes, **caractérisée en ce que** les cellules traitées ou non traitées sont des cultures primaires.

23. Méthode selon l'une quelconque des revendications précédentes, **caractérisée en ce que** les cellules traitées ou non traitées sont des cellules extraites de tissus or d'organes d'animaux traités ou non traités.

24. Méthode de diagnostic du potentiel toxique d'un composé test selon la revendication 1, comprenant au moins l'hybridation entre, d'une part, des sondes nucléiques marquées correspondant aux ARNm de cellules non traitées et une banque d'acides nucléiques comprenant des clones spécifiques de (portions de) gènes ou transcrits caractéristiques de situation d'apoptose et, d'autre part, des sondes nucléiques marquées correspondant aux ARNm de cellules traitées par ledit composé test et ladite banque d'acides nucléiques comprenant des clones spécifiques de (portions de) gènes ou transcrits caractéristiques de situation d'apoptose, le profil d'hybridation indiquant le potentiel toxique du composé test.

25. Méthode selon la revendication 24, **caractérisée en ce que** la banque comprend des clones spécifiques de (portions de) gènes ou transcrits caractéristiques de dérégulation de la voie p53.

26. Méthode selon la revendication 24 ou 25, **caractérisée en ce que** la banque d'acides nucléiques est une banque d'acides nucléiques caractéristiques de cellules transformées, en particulier de cellules tumorales.

27. Méthode selon la revendication 24, **caractérisée en ce que** la banque comprend au moins un clone de séquence choisie parmi SEQ ID Nos : 1 à 37, plus préférentiellement au moins 5.

28. Utilisation de clones d'acides nucléiques spécifiques de (portions de) gènes ou transcrits caractéristiques de situation d'apoptose comme marqueurs génétiques de toxicité de composés tests.

29. Utilisation selon la revendication 28, d'un clone de séquence choisie parmi SEQ ID Nos : 1 à 37,

30. Kit pour l'étude du potentiel toxique d'un composé test, comprenant un banque d'acides nucléiques comprenant au moins cinq clones de séquence choisie parmi SEQ ID Nos: 1 à 37, plus préférentiellement au moins 10.

31. Kit selon la revendication 30, **caractérisé en ce que** la banque est déposée sur un support.

32. Procédé de production de marqueurs génétiques de la toxicité, comprenant l'hybridation entre une population d'acides nucléiques dérivés de cellules en situation d'apoptose et une population d'acides nucléiques dérivés de cellules en situation de contrôle, l'isolement, à partir du produit de la réaction d'hybridation, de clones caractéristiques de la situation d'apoptose, et l'hybridation des clones obtenus avec un échantillon d'acides nucléiques dérivés de cellules en situation de toxicité.

33. Procédé de préparation d'une puce à ADN utilisable pour le diagnostic du potentiel toxique d'un composé test, comprenant le dépôt, sur un support solide, d'une ou plusieurs préparations d'acides nucléiques comprenant au moins 5 clones de séquence choisie parmi SEQ ID Nos: 1 à 37.

## Claims

1. A method of analysis of the toxic potential of a test compound, comprising at least one hybridization step between a) a nucleic acid sample from cells treated with this compound and b) a library of nucleic acids, said library comprising clones specific for (portions of) genes or transcripts which are characteristic of an apoptosis situation, the hybridization profile indicating the toxic potential of the test compound.

2. A method of analysis of the toxic potential of a test compound according to claim 1, comprising at least a separate hybridization between a) labelled nucleic probes corresponding to RNAs from untreated cells and from cells treated with said test compound and b) a library of nucleic acids, said library comprising clones specific for (portions of) genes or transcripts which are characteristic of an apoptosis situation, the hybridization profile indicating the toxic potential of the test compound.

3. Method according to claim 1 or 2, **characterized in that** the nucleic probes a) correspond to messenger RNAs from treated and untreated cells.

4. Method according to any one of claims 1 to 3, **characterized in that** the nucleic probes a) are cDNA or cDNA fragments prepared from the RNAs of treated and untreated cells.

5. Method according to any one of the previous claims, **characterized in that** the nucleic probes a) are amplification products.

6. Method according to any one of the previous claims, **characterized in that** the nucleic probes a) are labelled by radioactive, fluorescent, enzymatic or colorimetric labels.

7. Method according to any one of the previous claims, **characterized in that** the test compound is a compound of chemical or biological nature.

8. Method according to any one of the previous claims, **characterized in that** the library b) comprises nucleic acids corresponding to genes whose level of expression is modified in a situation of apoptosis.

9. Method according to one of claims 1 to 7, **characterized in that** the library b) comprises nucleic acids of which at least part of the sequence corresponds to the sequence of genes that are differentially spliced during an apoptosis situation.

10. Method according to one of claims 1 or 2, **characterized in that** the library b) comprises nucleic acids according to claim 8 and nucleic acids according to claim 9.

11. Method according to claim 9, **characterized in that** the library b) is prepared by hybridization between a nucleic acid population from a cell in a situation of cellular apoptosis and a nucleic acid population from a cell in a control situation, and isolating, from the hybrids formed, nucleic acids corresponding to differential splicings.

12. Method according to any one of the previous claims, **characterized in that** the situation of deregulation is induced by modification of the activation, preferably of the expression of a gene that initiates or carries out apoptosis.

13. Method according to claim 12, **characterized in that** the situation of deregulation is caused by induction or enhancement of the activation, preferably of the expression of an anti-oncogene.

14. Method according to claim 13, **characterized in that** the anti-oncogene is selected from p53, Rb, p73, myc, TUPRO-2 and NHTS.

15. Method according to claim 12, **characterized in that** the situation of deregulation is induced by modification of the activation, preferably of the expression of a gene involved in cell growth or viability.

16. Method according to claim 12, **characterized in that** the situation of deregulation is induced by activation, preferably constitutive or inducible expression of all or part of a gene involved in cell growth, cell viability or apoptosis.

17. Method according to any one of claims 1 to 7, wherein the library b) comprises at least 1 clone of sequence selected from SEQ ID Nos: 1 to 37, more preferably at least 5.

18. Method according to any one of claims 1 to 7, wherein the library b) comprises a set of probes, in particular 5 probes or more, preferably 10 probes or more, each of said probes being complementary to a part of a gene selected from the following genes : Aldolase A; S4 subunit of proteasome 26S ; Alpha-tubulin ; Glucosidase II ; lamin B receptor homologue; EF1-alpha ; Fra-1 ; tyrosine kinase AX1 receptor; spliceosomale Protein SAP62 ; TRAP-3 ; EF2 ; TEF-5 ; CDC25b ; interleukin-1 receptor-associated kinase (« IRAK ») ; WAF-1 ; c-fos (exon 4) ; ckshs1 ; PL16 ; NFAR-2; phosphatidylinositol4-kinase, ERF, Eph type receptor tyrosine kinase (hEphB1b) ; BAF60b protein of the SWI/SNF complex ; EB1 ; MSS1 ; retinoïc acid alpha receptor (RARa) ; translation initiation factor eiF4A; STE20 type kinase ; protein HSP 90kda ; Lipocortin II ; protein TPT1 (« translationally controlled tumor proteon »); Hsc70 ; Cytokeratin 18 ; 2-oxoglutarate dehydrogenase ; mitochondrial gene NADH6 ; mitochondrial gene NADH deshydrogenase 4 ; alpha subunit of mitochondrial ATP synthase.

19. Method according to any one of the previous claims, **characterized in that** the treated or untreated cells a) and the cells in a situation of deregulation b) are of a different type.

20. Method according to any one of the previous claims, **characterized in that** the treated or untreated cells are mammalian cells, preferably of human origin.

21. Method according to any one of the previous claims, **characterized in that** the treated or untreated cells are cell lines.

22. Method according to any one of the previous claims, **characterized in that** the treated or untreated cells are primary cultures.

23. Method according to any one of the previous claims, **characterized in that** the treated or untreated cells are cells extracted from organs or tissues of treated or untreated animals.

24. Method of diagnosis of the toxic potential of a test compound according to claim 1, comprising at least the hybridization between, on the one hand, labelled nucleic probes corresponding to mRNAs from untreated cells and a library of nucleic acids comprising clones specific for (portions of) genes or transcripts which are characteristic of an apoptosis situation and, on the other hand, labelled nucleic probes corresponding to mRNAs from cells treated with said test compound and said library of nucleic acids comprising clones specific for (portions of) genes or transcripts which are characteristic of an apoptosis situation, the hybridization profile indicating the toxic potential of the test compound.

25. Method according to claim 24, **characterized in that** the library comprises clones specific for (portions of) genes or transcripts which are characteristic of deregulation of the p53 pathway.

26. Method according to claim 24 or 25, **characterized in that** the library of nucleic acids is a library of nucleic acids characteristic of transformed cells, in particular of tumor cells.

27. Method according to claim 24, **characterized in that** the library of nucleic acids comprises at least 1 clone of sequence selected from SEQ ID Nos: 1 to 37, more preferably at least 5.

28. Use of nucleic acid clones specific for (portions of) genes or transcripts which are characteristic of an apoptosis situation, as genetic markers of toxicity of test compounds.

29. Use according to claim 28, of a clone of sequence selected from SEQ ID Nos: 1 to 37.

30. Kit for the study of the toxic potential of a test compound, comprising a library of nucleic acids comprising at least 5 clones of sequence selected from SEQ ID Nos: 1 to 37, more preferably at least 10.

31. Kit according to claim 30, wherein the bank is deposited on a support.

32. A process of production of genetic markers of toxicity, comprising hybridization between a nucleic acid population derived from cells in situation of apoptosis, and a nucleic acid population derived from cells in a control situation, the isolation, from the hybridization reaction product, of clones characteristic of the apoptosis situation, and the hybridization of the clones obtained with a nucleic acid sample derived from cells in a situation of toxicity.

33. A process of preparation of a DNA chip that can be used to diagnose the toxic potential of a test compound, comprising depositing, on a solid support, one or more nucleic acid preparations comprising at least 5 clones of a sequence selected from SEQ ID Nos: 1 to 37.

## Patentansprüche

1. Verfahren zur Untersuchung des toxischen Potenzials einer Testverbindung, umfassend wenigstens einen Hybridisierungsschritt von a) einer Probe an Nucleinsäuren von Zellen, die mit dieser Verbindung behandelt sind, und b) einer Bank an Nucleinsäuren, wobei besagte Bank Klone umfasst, die für charakteristische (Teile von) Gene(n) oder Transkripte des Apoptosezustandes spezifisch sind, und das Hybridisierungsmuster das toxische Potenzial der Testverbindung anzeigt.

2. Verfahren zur Untersuchung des toxischen Potenzials einer Testverbindung gemäß Anspruch 1, umfassend wenigstens eine separate Hybridisierung von a) markierten Nuclein-Sonden, die RNAs von nicht behandelten Zellen und mit besagter Testverbindung behandelten Zellen entsprechen, und b) einer Bank an Nucleinsäuren, wobei besagte Bank Klone umfasst, die für charakteristische (Teile von) Gene(n) oder Transkripte des Apoptosezustandes spezifisch sind, und das Hybridisierungsmuster das toxische Potenzial der Testverbindung anzeigt.

3. Verfahren gemäß Anspruch 1 oder 2, **dadurch gekennzeichnet, dass** die Nuclein-Sonden a) Messenger-RNAs aus behandelten und nicht behandelten Zellen entsprechen.

4. Verfahren gemäß einem der Ansprüche 1 bis 3, **dadurch gekennzeichnet, dass** die Nuclein-Sonden a) cDNA oder cDNA-Fragmente sind, die aus RNAs von behandelten oder nicht behandelten Zellen hergestellt sind.

5. Verfahren gemäß einem der vorhergehenden Ansprüche, **dadurch gekennzeichnet, dass** die Nuclein-Sonden a) Amplifikationsprodukte sind.

6. Verfahren gemäß einem der vorhergehenden Ansprüche, **dadurch gekennzeichnet, dass** die Nuclein-Sonden a) mit radioaktiven, fluoreszierenden, enzymatischen oder kolorimetrischen Markem markiert sind.

7. Verfahren gemäß einem der vorhergehenden Ansprüche, **dadurch gekennzeichnet, dass** die Testverbindung eine Verbindung chemischer oder biologischer Art ist.

8. Verfahren gemäß einem der vorhergehenden Ansprüche, **dadurch gekennzeichnet, dass** die Bank b) Nucleinsäuren umfasst, die Genen entsprechen, deren Expressionsniveau in einem Apoptosezustand modifiziert ist.

9. Verfahren gemäß einem der Ansprüche 1 bis 7, **dadurch gekennzeichnet, dass** die Bank b) Nucleinsäuren umfasst, deren Sequenz wenigstens zum Teil der Sequenz von Genen entspricht, die beim Apoptosezustand differentiell gespleißt sind.

10. Verfahren gemäß einem der Ansprüche 1 oder 2, **dadurch gekennzeichnet, dass** die Bank b) Nucleinsäuren gemäß Anspruch 8 und Nucleinsäuren gemäß Anspruch 9 umfasst.

11. Verfahren gemäß Anspruch 9, **dadurch gekennzeichnet, dass** die Bank b) hergestellt ist mittels Hybridisierung von einer Population an Nucleinsäuren von einer Zelle im zellulären Apoptosezustand und einer Population an Nucleinsäuren aus einer Zelle im Kontrollzustand und Isolieren aus den gebildeten Hybriden der Nucleinsäuren, die differentiellen Spleißungen entsprechen.

12. Verfahren gemäß einem der vorhergehenden Ansprüche, **dadurch gekennzeichnet, dass** der Deregulationszustand durch Modifikation der Aktivierung, bevorzugt der Expression eines die Apoptose initiierenden oder ausführenden Gens, induziert wird.

13. Verfahren gemäß Anspruch 12, **dadurch gekennzeichnet, dass** der Deregulationszustand durch Induktion oder Steigerung der Aktivierung, bevorzugt der Expression eines anti-onkogenen Gens, ausgelöst wird.

14. Verfahren gemäß Anspruch 13, **dadurch gekennzeichnet, dass** das anti-onkogene Gen aus p53, Rb, p73, myc, TUPRO-2 und NHTS ausgewählt ist.

15. Verfahren gemäß Anspruch 12, **dadurch gekennzeichnet, dass** der Deregulationszustand durch Modifikation der Aktivierung, bevorzugt der Expression eines Gens induziert ist, das am Zellwachstum oder an der Zelllebensfähigkeit beteiligt ist.

16. Verfahren gemäß Anspruch 12, **dadurch gekennzeichnet, dass** der Deregulationszustand durch Aktivierung bevorzugt der konstitutiven oder induzierbaren Expression des ganzen oder eines Teils eines Genes induziert wird, das am Zellwachstum, an der Zelllebensfähigkeit oder an der Apoptose beteiligt ist.

17. Verfahren gemäß einem der Ansprüche 1 bis 7, **dadurch gekennzeichnet, dass** die Bank b) wenigstens einen Klon, bevorzugter wenigstens 5 Klone, mit der aus der SEQ ID NR: 1 bis 37 ausgewählten Sequenz umfasst.

18. Verfahren gemäß einem der Ansprüche 1 bis 7, **dadurch gekennzeichnet, dass** die Bank b) eine Gruppe Sonden, besonders 5 Sonden oder mehr, bevorzugt 10 Sonden oder mehr, umfasst, wobei jede Sonde zu einem Teil eines Genes komplementär ist, das aus den Genen ausgewählt ist: Aldolase A; S4-Untereinheit des 26S Proteasoms; Alpha-Tubulin; Glucosidase II; Lamin B Rezeptor-Homolog; EF1-alpha; Fra-1, Rezeptor von Tyrosin-Kinase AX1; spleißomales SAP62 Protein; TRAF-3; EF2; TEF-5; CDC25b, Interieukin-1 Rezeptor assoziierte Kinase («IRAK»); WAF-1; c-fos (Exon 4); ckshs1; PL16; NFAR-2; Phosphatidylinositol4-Kinase; ERF; Tyrosin-Kinase vom Rezeptor-Typ Eph (hEphB1b); BAF60b Protein des SWI/SNF-Komplexes; EB1; MSS1; alpha-Rezeptor der Retinoinsäure RARa); Translations-Initiationsfaktor eIF4A; Kinase vom Typ STE20; 90 kDa HSP-Protein,; Lipocortin II; TPT1 Protein («translationally controlled tumor proteon»); Hsc70; Cytokeratin 18; 2-Oxoglutarat-Dehydrogenase; mitochondriales NADH6-Gen; mitochondriales NADH Dehydrogenase 4 Gen; alpha-Untereinheit der mitochondrialen ATP-Synthase.

19. Verfahren gemäß einem der vorhergehenden Ansprüche, **dadurch gekennzeichnet, dass** die behandelten oder nicht behandelten Zellen a) und im Deregulationszustand b) unterschiedlichen Typs sind.

20. Verfahren gemäß einem der vorhergehenden Ansprüche, **dadurch gekennzeichnet, dass** die behandelten oder nicht behandelten Zellen Säugerzellen vorzugsweise Humanzellen sind.

21. Verfahren gemäß einem der vorhergehenden Ansprüche, **dadurch gekennzeichnet, dass** die behandelten oder nicht behandelten Zellen Zelllinien sind.

22. Verfahren gemäß einem der vorhergehenden Ansprüche, **dadurch gekennzeichnet, dass** die behandelten oder nicht behandelten Zellen Primärkulturen sind.

23. Verfahren gemäß einem der vorhergehenden Ansprüche, **dadurch gekennzeichnet, dass** die behandelten oder nicht behandelten Zellen Zellextrakte von Geweben oder Organen behandelter oder nicht behandelter Tiere sind.

24. Verfahren zur Diagnose des toxischen Potenzials einer Testverbindung gemäß Anspruch 1, umfassend wenigstens die Hybridisierung einerseits von markierten Nuclein-Sonden, die mRNAs von nicht behandelten Zellen entsprechen, und einer Bank an Nucleinsäuren, umfassend Klone, die für charakteristische (Teile von) Gene(n) oder Transkripte des Apoptosezustandes spezifisch sind, und andererseits von markierten Nuclein-Sonden, die mRNAs von mit besagter Testverbindung behandelten Zellen entsprechen, und besagter Bank an Nucleinsäuren, umfassend Klone, die für charakteristische (Teile von) Gene(n) oder Transkripte des Apoptosezustandes spezifisch sind, wobei das Hybridisierungsmuster das toxische Potenzial der Testverbindung anzeigt.

25. Verfahren gemäß Anspruch 24, **dadurch gekennzeichnet, dass** die Bank Klone umfasst, die für charakteristische (Teile von) Gene(n) oder Transkripte der Deregulation des p53 Pfades spezifisch sind.

26. Verfahren gemäß Anspruch 24 oder 25, **dadurch gekennzeichnet, dass** die Nucleinsäure-Bank eine für transformierte Zellen, besonders Tumorzellen, charakteristische Nucleinsäure-Bank ist.

27. Verfahren gemäß Anspruch 24, **dadurch gekennzeichnet, dass** die Bank wenigstens einen Klon, bevorzugter wenigstens 5 Klone, mit der aus den SEQ ID NR: 1 bis 37 ausgewählten Sequenz umfasst.

28. Verwendung von Nucleinsäure-Klonen, die für charakteristische (Teile von) Gene(n) oder Transkripte des Apoptosezustandes spezifisch sind, als Genmarker der Toxizität von Testverbindungen.

29. Verwendung eines Klons gemäß Anspruch 28 mit der aus den SEQ ID NR: 1 bis 37 ausgewählten Sequenz.

30. Kit zur Untersuchung des toxischen Potenzials einer Testverbindung, umfassend eine Bank an Nucleinsäuren, umfassend wenigstens 5 Klone, bevorzugter wenigstens 10 Klone, mit der aus den SEQ ID NR: 1 bis 37 ausgewählten Sequenz.

31. Kit gemäß Anspruch 30, **dadurch gekennzeichnet, dass** die Bank auf einem Träger angebracht ist.

32. Verfahren zur Herstellung von Genmarkem der Toxizität, umfassend die Hybridisierung von einer Population an Nucleinsäuren, die von Zellen im Apoptosezustand stammen, und einer Population an Nucleinsäuren, die von Zellen im Kontrollzustand stammen, Isolieren aus dem Produkt der Hybridisierungsreaktion von Klonen, die für den Apoptose-Zustand charakteristisch sind, und Hybridisierung von Klonen, die mit einer von Zellen im Toxizitätszustand stammenden Nucleinsäure-Probe erhalten sind.

33. Verfahren zur Herstellung eines DNA-Chips, der für die Diagnose eines toxischen Potenzials einer Testverbindung einsetzbar ist, umfassend die Anlagerung einer oder mehrerer Nucleinsäure-Präparate auf einem Träger, umfassend wenigstens 5 Klone mit der aus den SEQ ID NR: 1 bis 37 ausgewählten Sequenz.
